(19) Europäisches Patentamt
European Patent Office
Office européen des brevets

(11) **EP 0 729 302 B1**

(12) **EUROPEAN PATENT SPECIFICATION**

(45) Date of publication and mention
of the grant of the patent:
**08.03.2000 Bulletin 2000/10**

(51) Int. Cl.[7]: **A01N 59/16**, A61L 2/16,
C23C 14/14

(21) Application number: **94931472.8**

(22) Date of filing: **01.11.1994**

(86) International application number:
**PCT/CA94/00604**

(87) International publication number:
**WO 95/13704 (26.05.1995 Gazette 1995/22)**

(54) **ANTI-MICROBIAL MATERIALS**

ANTIMIKROBIELLE MATERIALEN

PRODUITS ANTIMICROBIENS

(84) Designated Contracting States:
**AT BE CH DE DK ES FR GB GR IE IT LI LU MC NL
PT SE**
Designated Extension States:
**LT SI**

(30) Priority: **18.11.1993 US 154490
18.11.1993 US 154693
18.11.1993 US 154694
02.02.1994 US 190617**

(43) Date of publication of application:
**04.09.1996 Bulletin 1996/36**

(60) Divisional application:
**98103781.5 / 0 875 146**

(73) Proprietor:
**WESTAIM TECHNOLOGIES INC.
Fort Saskatchewan, Alberta T8L 2P2 (CA)**

(72) Inventors:
• **BURRELL, Robert, Edward
Sherwood Park, Alberta T8A 4L6 (CA)**

• **APTE, Prasad, Shrikrishna
St. Albert, Alberta T8N 3W1 (CA)**
• **GILL, Kashmir, Singh
Sherwood Park, Alberta T8A 3W1 (CA)**
• **PRECHT, Roderick, John
Edmonton, Alberta T6K 3S7 (CA)**
• **MORRIS, Larry, Roy
Yarker, Ontario K0K 3N0 (CA)**
• **MCINTOSH, Catherine, Laurie
Sherwood Park, Alberta T8A 2T7 (CA)**
• **SANT, Sudhindra, Bharat
Edmonton, Alberta T5Y 1Z4 (CA)**

(74) Representative:
**Hedley, Nicholas James Matthew
Stephenson Harwood
One, St. Paul's Churchyard
London EC4M 8SH (GB)**

(56) References cited:
**EP-A- 0 488 269** **WO-A-93/23092**
**DE-A- 2 530 487** **DE-C- 819 131**
**DE-U- 9 017 361** **FR-A- 2 634 986**
**GB-A- 2 073 024** **GB-A- 2 134 791**
**US-A- 2 103 999**

EP 0 729 302 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

**FIELD OF THE INVENTION**

The invention relates to methods of forming anti-microbial metal coatings, foils and powders which provide a sustained release of anti-microbial metal species when in contact with an alcohol or electrolyte.

**BACKGROUND OF THE INVENTION**

The need for an effective anti-microbial coating is well established in the medical community. Physicians and surgeons using medical devices and appliances ranging from orthopaedic pins, plates and implants through to wound dressings and urinary catheters must constantly guard against infection. An inexpensive anti-microbial coating also finds application in medical devices used in consumer healthcare and personal hygiene products as well as in biomedical/biotechnical laboratory equipment The term "medical device", as used herein and in the claims is meant to extend to all such products.

The anti-microbial effects of metallic ions such as Ag, Au, Pt, Pd, Ir (i.e. the noble metals), Cu, Sn, Sb, Bi and Zn are known (see Morton, H.E., Pseudomonas in Disinfection, Sterilization and Preservation, ed. S.S. Block, Lea and Febiger, 1977 and Grier, N., Silver and Its Compounds in Disinfection, Sterilization and Preservation, ed. S.S. Block, Lea and Febiger, 1977). Of the metallic ions with anti-microbial properties, silver is perhaps the best known due to its unusually good bioactivity at low concentrations. This phenomena is termed oligodynamic action. In modern medical practice both inorganic and organic soluble salts of silver are used to prevent and treat microbial infections. While these compounds are effective as soluble salts, they do not provide prolonged protection due to loss through removal or complexation of the free silver ions. They must be reapplied at frequent intervals to overcome this problem. Reapplication is not always practical, especially where an in-dwelling or implanted medical device is involved.

Attempts have been make to slow the release of silver ions during treatment by creating silver containing complexes which have a lower level of solubility. For example, U.S. Patent 2,785,153 discloses colloidal silver protein for this purpose. Such compounds are usually formulated as creams. These compounds have not found wide applicability in the medical area due to their limited efficacy. The silver ion release rate is very slow. Furthermore, coatings from such compounds have been limited due to adhesion, abrasion resistance and shelf life problems.

The use of silver metal coatings for anti-microbial purposes has been suggested. For instance, see Deitch et al., Anti-microbial Agents and Chemotherapy, Vol. 23(3), 1983, pp. 356 - 359 and Mackeen et al., Anti-microbial Agents and Chemotherapy, Vol. 31(1), 1987. pp. 93 - 99. However, it is generally accepted that such coatings alone do not provide the required level of efficacy, since diffusion of silver ions from the metallic surface is negligible.

A silver metal coating is produced by Spire Corporation, U.S.A. under the trade mark SPI-ARGENT. The coating is formed by an ion-beam assisted deposition (IBAD) coating process. The infection resistant coating is stated to be non-leaching in aqueous solutions as demonstrated by zone of inhibition tests, thus enforcing the belief that silver metal surfaces do not release anti-microbial amounts of silver ions.

Given the failure of metallic silver coatings to generate the required anti-microbial efficacy, other researchers have tried novel activation processes. One technique is to use electrical activation of metallic silver implants (see Marino et al., Journal of Biological Physics, Vol. 12, 1984, pp. 93 - 98). Electrical stimulation of metallic silver is not always practical, especially for mobile patients. Attempts to overcome this problem include developing in situ electrical currents through galvanic action. Metal bands or layers of different metals are deposited on a device as thin film coatings. A galvanic cell is created when two metals in contact with each other are placed in an electrically conducting fluid. One metal layer acts as an anode, which dissolves into the electrolyte. The second metal acts as a cathode to drive the electrochemical cell. For example, in the case of alternating layers of Cu and Ag, the Cu is the anode, releasing $Cu^+$ ions into the electrolyte. The more noble of the metals, Ag, acts as the cathode, which does not ionize and does not go into solution to any large extent. An exemplary device of this nature is described in U.S. Patent 4,886,505 issued Dec. 12, 1989, to Haynes et al. The patent discloses sputtered coatings of two or more different metals with a switch affixed to one of the metals such that, when the switch is closed, metal ion release is achieved.

Previous work has shown that a film composed of thin laminates of alternating, different metals such as silver and copper can be made to dissolve if the surface is first etched. In this instance, the etching process creates a highly textured surface (see M. Tanemura and F. Okuyama, J. Vac. Sci. Technol., 5, 1986, pp 2369-2372). However, the process of making such multilaminated films is time consuming and expensive.

Electrical activation of metallic coatings has not presented a suitable solution to the problem. It should be noted that galvanic action will occur only when an electrolyte is present and if an electrical connection between the two metals of the galvanic couple exists. Since galvanic corrosion occurs primarily at the metallic interface between the two metals, electrical contact is not sustained. Thus a continuous release of metal ions over an extended period of time is not probable. Also, galvanic action to release a metal such as silver is difficult to achieve. As indicated above, the metal ions exhibiting the greatest anti-microbial effect are the noble metals, such as Ag, Au, Pt and Pd. There are few metals more noble than these to serve as cathode materials so as to drive the release of a noble metal such as Ag at the anode.

A second approach to activating the silver metal surface is to use heat or chemicals. U.S. Patents 4,476,590 and 4,615,705, issued to Scales et al. on October 16, 1984 and October 7, 1986, respectively, disclose methods of activating silver surface coatings on endoprosthetic implants to render them bioerodible by heating at greater than 180°C or by contacting with hydrogen peroxide. Such treatments are limited in terms of the substrate/devices which can be coated and activated.

There is still a need for an efficacious, inexpensive anti-microbial material having the following properties:

- sustained release of an anti-microbial agent at therapeutically active levels;
- applicable to a wide variety of devices and materials;
- useful shelf life; and
- low mammalian toxicity.

Metal coatings are typically produced as thin films by vapour deposition techniques such as sputtering. Thin films of metals, alloys, semiconductors and ceramics are widely used in the production of electronic components. These and other end uses require the thin films to be produced as dense, crystalline structures with minimal defects. The films are often annealed after deposition to enhance grain growth and recrystallization and produce stable properties. Techniques to deposit metal films are reviewed by R.F. Bunshah et al., "Deposition Technologies for Films and Coatings", Noyes Publications, N.J., 1982 and by J.A. Thornton, "Influence of Apparatus Geometry and Deposition Conditions on the Structure and Topography or Thick Sputtered Coatings", J. Vac. Sci. Technol., 11(4), 666-670, 1974.

U.S. Patent No. 4,325,776, issued April 20, 1982 to Menzel discloses a process for producing coarse or single crystal metal films from certain metals for use in integrated circuits. The metal film is formed by depositing on a cooled substrate (below -90°C) such that the metal layer is in an amorphous phase. The metal layer is then annealed by heating the substrate up to about room temperature. The end product is stared to have large grain diameter and great homogeneity, permitting higher current densities without electromigration failures.

Silver salts such as those of nitrate, proteins, acetate, lactate and citrate have been suggested for use in anti-microbial coatings for medical devices. Silver nitrate is used in burn wound dressings in many hospitals. These salts are known to have better anti-microbial efficacy than silver metal. The mechanism by which these compounds are effective is the instant ionization/dissociation to produce the $Ag^+$ ion. The availability of the $Ag^+$ ion is reduced significantly within or in contact with bodily fluids or tissues. Due to the high chloride content of such fluids, the silver is precipitated or tied up as insoluble silver chloride ($Ksp = 1.7 \times 10^{-10}M$). As a consequence, excessive amounts of silver must be present within any media containing precipitants (chiefly chloride) in order to produce the same efficacy from a silver salt as would be observed in water.

Nanocrystalline materials in the forms of powders, films and flakes are materials which are single-phase or multi-phase polycrystals, the grain size of which is in the order of a few (typically <20) nanometers in at least one dimension. Fine grain powders (particle size <5 microns) may be nanocrystalline, or more typically have grain sizes >20 nm. Nanocrystalline materials and fine powders may be prepared by a number of modified gas condensation methods, wherein the material to be deposited is generated in the vapour phase, for example by evaporation or sputtering, and is transported into a relatively large volume in which the working gas atmosphere and temperature is controlled. Atoms of the material to be deposited collide with atoms of the working gas atmosphere, lose energy and are rapidly condensed from the vapour phase onto a cold substrate, such as a liquid nitrogen cooled finger. In principle, any method capable of producing very fine grain sized polycrystalline materials can be used to produce nanocrystalline materials. These methods include, for example, evaporation such as arc evaporation, electron beam vapor deposition, molecular beam epitaxy, ion beam, sputtering, magnetron sputtering and reactive sputtering (see for example, Froes, F.H. et al., "Nanocrystalline Metals for Structural Applications", JOM, 41(1989), No. 6., pp 12 - 17; Birringer, Rainer et al., "Nanocrystalline Materials - A First Report, Proceedings of JIMIS-4; and Gleiter, H. "Materials with Ultrafine Microstructures: Retrospectives and Perspectives, NanoStructured Materials, Vol. 1, pp 1-19, 1992, and references cited therein).

FR-A-2 634 986 discloses artificial hair fibres coated with amorphous silver and having an anti-microbial effect.

GB-A-2 134 791 discloses anti microbial surgical dressings formed by deposition onto dried moss.

DE-U-9017361 discloses containers for liquid medicaments containing anti-microbial amorphous metal.

WO-A-9323092 discloses anti-microbial coatings and powders containing atomic disorder to release anti-microbial atoms, ions, molecules or clusters.

SUMMARY OF THE INVENTION

The inventors set out to develop an anti-microbial metal coating. They discovered that, contrary to previous belief, it is possible to form metal coatings from an anti-microbial metal material by creating atomic disorder in the materials by vapour deposition under conditions which limit diffusion, that is which "freeze-in" the atomic disorder. The anti-microbial coatings so produced were found to provide sustained release of anti-microbial metal species into solution so as to produce an anti-microbial effect.

This basic discovery linking "atomic disorder" to enhanced solubility has broad application. The inventors have demonstrated that atomic disorder so as to produce solubility can be created in other material forms, such as metal powders. The invention also has application beyond anti-microbial metals, encompassing any metal, metal alloy, or metal compound, including semiconductor or ceramic materials, from which sustained release of metal species into solution is desired. For instance, materials having enhanced or controlled metal dissolution find application in sensors, switches, fuses, electrodes, and batteries.

The term "atomic disorder" as used herein includes high concentrations of: point defects in a crystal lattice, vacancies, line defects such as dislocations, interstitial atoms, amorphous regions, grain and sub grain boundaries and the like relative to its normal ordered crystalline state. Atomic disorder leads to irregularities in surface topography and inhomogeneities in the structure on a nanometre scale.

By the term "normal ordered crystalline state" as used herein is meant the crystallinity normally found in bulk metal materials, alloys or compounds formed as cast, wrought or plated metal products. Such materials contain only low concentrations of such atomic defects as vacancies, grain boundaries and dislocations.

The term "diffusion" as used herein implies diffusion of atoms and/or molecules on the surface or in the matrix of the material being formed.

The terms "metal" or "metals" as used herein are meant to include one or more metals whether in the form of substantially pure metals, alloys or compounds such as oxides, nitrides, borides, sulphides, halides or hydrides.

The invention, in a broad aspect extends to a method of forming an anti-microbial material containing one or more metals as set out in accompanying claims 1 to 16 and 28 to 33. The method comprises creating atomic disorder in the material such that sufficient atomic disorder is retained in the material to provide release, on a sustainable basis, of atoms, ions, molecules or clusters of at least one of the metals into an alcohol or water-based electrolyte. Clusters are known to be small groups of atoms, ions or the like, as described by R.P. Andres et al., "Research Opportunities on Clusters and Cluster-Assembled Materials", J. Mater. Res. Vol. 4, No. 3, 1989, P. 704.

Specific preferred embodiments of the invention demonstrate that atomic disorder may be created in metal powders or foils by cold working, and in metal coatings by depositing by vapour deposition at low substrate temperatures.

In preferred embodiments of the invention, the modified material is a metal powder which has been mechanically worked or compressed, under cold working conditions, to create and retain atomic disorder.

The term "metal powder" as used herein is meant to include metal particles of a broad particle size, ranging from nanocrystalline powders to flakes.

The term "cold working" as used herein indicates that the material has been mechanically worked such as by milling, grinding, hammering, mortar and pestle or compressing, at temperatures lower than the recrystallization temperature of the material. This ensures that atomic disorder imparted through working is retained in the material.

In another preferred embodiment, the modified material is a metal coating formed on a substrate by vapour deposition techniques such as vacuum evaporation, sputtering, magnetron sputtering or ion plating. The material is formed under conditions which limit diffusion during deposition and which limit annealing or recrystallization following deposition. The deposition conditions preferably used to produce atomic disorder in the coatings are outside the normal range of operating conditions used to produce defect free, dense, smooth films. Such normal practices are well known (see for example R.F. Bunshah er al., supra.) Preferably the deposition is conducted at low substrate temperatures such that the ratio of the substrate temperature to the melting point of the metal or metal compound being deposited (T/Tm) is maintained at less than about 0.5, more preferably at less than about 0.35, and most preferably at less than 0.30. In this ratio, the temperatures are in degrees Kelvin. The preferred ratio will vary from metal to metal and increases with alloy or impurity content. Other preferred deposition conditions to create atomic disorder include one or more of a higher than normal working gas pressure, a lower than normal angle of incidence of the coating flux and a higher than normal coating flux.

The temperature of deposition or cold working is not so low that substantial annealing or recrystallization will take place when the material is brought to room temperature or its intended temperature for use (ex. body temperature for anti-microbial materials). If the temperature differential between deposition and temperature of use ($\Delta T$) is too great, annealing results, removing atomic disorder. This $\Delta T$ will vary from metal to metal and with the deposition technique used. For example, with respect to silver, substrate temperatures of -20 to 200°C arc preferred during physical vapour deposition.

Normal or ambient working gas pressure for depositing the usually required dense, smooth, defect free metal films vary according to the method of physical vapour deposition being used. In general, for sputtering, the normal working gas pressure is less than 10 Pa (Pascal) (75 mT (milliTorr)), for magnetron sputtering, less than 1.3 Pa (10mT), and for ion-plating less than 30 Pa (200 mT). Normal ambient gas pressures for vacuum evaporation processes vary as follows: for e-beam or arc evaporation, from 0.0001 Pa (0.001 mT) to 0.001 Pa (0.01 mT); for gas scattering evaporation (pressure plating) and reactive arc evaporation, up to 30 Pa (200 mT), but typically less than 3 Pa (20 mT). Thus, in accordance with the method of the present invention, in addition to using low substrate temperatures to achieve atomic disorder, working (or ambient) gas pressures higher than these normal values may be used to increase the level of atomic disorder in the coating.

Another condition discovered to have an effect on the level of atomic disorder in the coatings of the present invention is the angle of incidence of the coating flux during deposition. Normally to achieve dense, smooth coatings, this angle is maintained at about 90° +/- 15°. In accordance with the present invention, in addition to using low substrate temperatures during deposition to achieve atomic disorder, angles of incidence lower than about 75° may be used to increase the level of atomic disorder in the coating.

Yet another process parameter having an effect on the level of atomic disorder is the atom flux to the surface being coated. High deposition rates tend to increase atomic disorder, however, high deposition rates also tend to increase the coating temperature. Thus, there is an optimum deposition rate that depends on the deposition technique, the coating material and other process parameters.

To provide an anti-microbial material, the metals used in the coating or powder are those which have an anti-microbial effect, but which are biocompatible (non-toxic for the intended utility). Preferred metals include Ag, Au, Pt, Pd, Ir (i.e. the noble metals), Sn, Cu, Sb, Bi, and Zn, compounds of these metals or alloys containing one or more of these metals. Such metals are hereinafter referred to as "anti-microbial metals"). Most preferred is Ag or its alloys and compounds. Anti-microbial materials in accordance with this invention preferably are formed with sufficient atomic disorder that atoms, ions, molecules or clusters of the anti-microbial material are released into an alcohol or water based electrolyte on a sustainable basis. The term "sustainable basis" is used herein to differentiate, on the one hand from the release obtained from bulk metals, which release metal ions and the like at a rate and concentration which is too low to achieve an anti-microbial effect, and on the other hand from the release obtained from highly soluble salts such as silver nitrate, which release silver ions virtually instantly in contact with an alcohol or water based electrolyte. In contrast, the anti-microbial materials of the present invention release atoms, ions, molecules or clusters of the anti-microbial metal at a sufficient rate and concentration, over a sufficient time period to provide a useful anti-microbial effect.

The term "anti-microbial effect" as used herein means that atoms, ions, molecules or clusters of the anti-microbial metal are released into the electrolyte which the material contacts in concentrations sufficient to inhibit bacterial growth in the vicinity of the material. The most common method of measuring anti-microbial effect is by measuring the zone of inhibition (ZOI) created when the material is placed on a bacterial lawn. A relatively small or no ZOI (ex. less than 1 mm) indicates a non-useful anti-microbial effect, while a larger ZOI (ex. greater than 5 mm) indicates a highly useful anti-microbial effect. One procedure for a ZOI test is set out in the Examples which follow.

The invention extends to devices such as medical devices formed from, incorporating, carrying or coated with the anti-microbial powders or coatings. The anti-microbial coating may be directly deposited by vapour deposition onto such medical devices as catheters, sutures, implants, burn dressings and the like. An adhesion layer, such as tantalum, may be applied between the device and the anti-microbial coating. Adhesion may also be enhanced by methods known in the art, for example etching the substrate or forming a mixed interface between the substrate and the coating by simultaneous sputtering and etching. Anti-microbial powders may be incorporated into creams, polymers, ceramics, paints, or other matrices, by techniques well known in the art.

In accordance with the invention, modified materials may be prepared as composite metal coatings containing atomic disorder. In this case, the coating of the one or more metals or compounds to be released into solution constitutes a matrix containing atoms or molecules of a different material. The presence of different atoms or molecules results in atomic disorder in the metal matrix, for instance due to different sized atoms. The different atoms or molecules may be one or more second metals, metal alloys, or metal compounds which are co- or sequentially deposited with the first metal or metals to be released. Alternatively the different atoms or molecules may be absorbed or trapped from the working gas atmosphere during reactive vapour deposition. The degree of atomic disorder, and thus solubility, achieved by the inclusion of the different atoms or molecules varies, depending on the materials. In order to retain and enhance the atomic disorder in the composite material, one or more of the above-described vapour deposition conditions, namely low substrate temperature, high working gas pressure, low angle of incidence and high coating flux, may be used in combination with the inclusion of different atoms or molecules.

Preferred composite materials for anti-microbial purposes are formed by including atoms or molecules containing oxygen, nitrogen, hydrogen, boron, sulphur or halogens in the working gas atmosphere while depositing the anti-microbial metal. These atoms or molecules are incorporated in the coating either by being absorbed or trapped in the film, or by reacting with the metal being deposited. Both of these mechanisms during deposition are hereinafter referred to as "reactive deposition". Gases containing these elements, for example oxygen, hydrogen, and water vapour, may be provided continuously or may be pulsed for sequential deposition.

Anti-microbial composite materials are also preferably prepared by co- or sequentially depositing an anti-microbial metal with one or more inert biocompatible metals selected from Ta, Ti, Nb, Zn, V, Hf, Mo, Si, and Al. Alternatively, the composite materials may be formed by co-, sequentially or reactively depositing one or more of the anti-microbial metals as the oxides, carbides, nitrides, borides, sulphides or halides of these metals and/or the oxides, carbides, nitrides, borides, sulphides or halides of the inert metals. Particularly preferred composites contain oxides of silver and/or gold, alone or together with one or more oxides of Ta, Ti, Zn and Nb.

The invention provides the activating or further enhancing the anti-microbial effect of anti-microbial materials formed with atomic disorder by, irradiation to further enhance the anti-microbial effect. However, it is also possible to irradiate materials initially formed with a level of atomic disorder which is insufficient to produce an anti-microbial effect, such that the irradiated material has an acceptable anti-microbial effect. The process of activation comprises irradiating the material with a low linear energy transfer form of radiation such as beta or x-rays, but most preferably gamma rays. A dose greater than 1 Mrad is preferred. The anti-microbial material is preferably oriented substantially perpendicular to the incoming radiation. The level of activation may be further enhanced by irradiating the material adjacent to a dielectric material such as oxides of Ta, Al and Ti, but most preferably silicon oxide.

The invention further extends to fine grain anti-microbial materials, comprising one or more anti-microbial metals or alloys or compounds thereof, having a grain size less than 200 nm, in a fine crystalline powder form, as set out in accompanying claims 17 to 23.

The anti-microbial material may be prepared by introducing the atomic disorder into a pre-formed fine grain or nanocrystalline (<20 nm) powder, flakes or films of one or more of the anti-microbial metals by mechanical working, for example by compressing the material, under cold working conditions. Alternatively, the atomic disorder may be created during the synthesis of fine grain or nanocrystalline materials (films, flakes or powders) by vapour deposition techniques in which the anti-microbial metal is co-, sequentially or reactively deposited in a matrix with atoms or molecules of a different material under conditions such that atomic disorder is created and retained in the matrix. The different material (or dopant) is selected from inert biocompatible metals, oxygen, nitrogen, hydrogen, boron, sulphur, and halogens, and oxides, nitrides, carbides, borides, sulphides and halides of either or both of an anti-microbial metal or a biocompatible metal. Preferred biocompatible metals include Ta, Ti, Nb, B, Hf, Zn, Mo, Si, and Al. These different materials may be included with the anti-microbial metal in the same or separate target, for example a target of Ag and/or silver oxides, which may further contain, for example, Ta or tantalum oxides. Alternatively, the different material may be introduced from the working gas atmosphere during vapour deposition, for example by sputtering or reactive sputtering in an atmosphere containing atoms or molecules of the different material such as oxygen.

The anti-microbial form of silver material prepared in accordance with the process of the present invention has been physically characterized and has been found to have the following novel characteristics:

- a positive rest potential, $E_{rest}$ When measured against a saturated calomel reference electrode (SCE), in 1 M potassium hydroxide;
- preferably a ratio of temperature of recrystallization to its melting point, in degrees K, $(T_{rest}/T_m)$, of less than about 0.33, and most preferably less than about 0.30;
- preferably a temperature of recrystallization less than about 140 °C; and
- preferably, a grain size less than about 200nm, preferably less than 140 nm and most preferably less than 90 nm.

Each of these physical characteristics, with perhaps the exception of grain size, is believed to be the result of the presence of atomic disorder in the material. The characteristics are of assistance in identifying and distinguishing the silver materials of the present invention from prior art materials or materials in their normal ordered crystalline state. The preferred novel anti-microbial silver materials have been characterized, for example by XRD, XPS and SIMS analysis, as comprising substantially pure silver metal, when deposited in an inert atmosphere such as argon. However, when the working gas atmosphere contains oxygen, the materials comprise a matrix of substantially pure silver metal and one or both of, silver oxide and atoms or molecules of trapped or absorbed oxygen. A further distinguishing feature of the materials of the present invention is the presence of growth twins in the grain structure, visible from TEM analysis.

## BRIEF DESCRIPTION OF THE DRAWINGS

Figure 1 is a TEM micrograph of a sputter deposited silver coating in accordance with the invention, illustrating grain size and growth twin defects.

Figure 2 is a TEM micrograph of the film of Figure 1 after annealing, showing larger grain size and the presence of annealing twins.

## DESCRIPTION OF THE PREFERRED EMBODIMENTS

As above stated, the present invention has application beyond anti-microbial materials. However, the invention is disclosed herein with anti-microbial metals, which are illustrative of utility for other metals, metal alloys and metal compounds. Preferred metals include Al and Si, and the metal elements from the following groups of the periodic table: IIIB, IVB, VB, VIB, VIIB, VIIIB, IB, IIB, IIIA, IVA, and VA (excluding As) in the periods 4, 5 and 6, (see Periodic Table as published in Merck Index 10th Ed., 1983, Merck and Co. Inc., Rahway, N.J., Martha Windholz). Different metals will have varying degrees of solubility. However, the creation and retention of atomic disorder in accordance with this invention results in enhanced solubility (release) of the metal as ions, atoms, molecules or clusters into an appropriate solvent i.e. a solvent for the particular material, typically a polar solvent, over the solubility of the material in its normal ordered crystalline state.

The medical devices formed from, incorporating, carrying or coated with the anti-microbial material of this invention generally come into contact with an alcohol or water based electrolyte including a body fluid (for example blood, urine or saliva) or body tissue (for example skin, muscle or bone) for any period of time such that microorganism growth on the device surface is possible. The term "alcohol or water based electrolyte" also includes alcohol or water based gels. In most cases the devices are medical devices such as catheters, implants, tracheal tubes, orthopaedic pins, insulin pumps, wound closures, drains, dressings, shunts, connectors, prosthetic devices, pacemaker leads, needles, surgical instruments, dental prostheses, ventilator tubes and the like. However, it should be understood that the invention is not limited to such devices and may extend to other devices useful in consumer healthcare, such as sterile packaging, clothing and footwear, personal hygiene products such as diapers and sanitary pads, in biomedical or biotechnical laboratory equipment, such as tables, enclosures and wall coverings, and the like. The term "medical device" as used herein and in the claims is intended to extend broadly to all such devices.

The device may be made of any suitable material, for example metals, including steel, aluminum and its alloys, latex, nylon, silicone, polyester, glass, ceramic, paper, cloth and other plastics and rubbers. For use as an in-dwelling medical device, the device will be made of a bioinert material. The device may take on any shape dictated by its utility, ranging from flat sheets to discs, rods and hollow tubes. The device may be rigid or flexible, a factor again dictated by its intended use.

Anti-Microbial Coatings

The and-microbial coating in accordance with this invention is deposited as a thin metallic film on one or more surfaces of a medical device by vapour deposition techniques. Physical vapour techniques, which are well known in the art, all deposit the metal from the vapour, generally atom by atom, onto a substrate surface. The techniques include vacuum or arc evaporation, sputtering, magnetron sputtering and ion plating. The deposition is conducted in a manner to create atomic disorder in the coating as defined hereinabove. Various conditions responsible for producing atomic disorder are useful. These conditions are generally avoided in thin film deposition techniques where the object is to create a defect free, smooth and dense film (see for example J.A. Thornton, supra). While such conditions have been investigated in the art, they have not heretofore been linked to enhanced solubility of the coatings so-produced.

The preferred conditions which we used to create atomic disorder during the deposition process include:

- a low substrate temperature, that is maintaining the surface to be coated at a temperature such that the ratio of the substrate temperature to the melting point of the metal (in degrees Kelvin) is less than about 0.5, more preferably less than about 0.35 and most preferably less than about 0.3; and optionally one or both of:
- a higher than normal working (or ambient) gas pressure, i.e. for vacuum evaporation: e-beam or arc evaporation, greater than 0.001 Pa (0.01 mT), gas scattering evaporation (pressure plating) or reactive arc evaporation, greater than 3 Pa (20 mT); for sputtering: greater than 10 Pa (75 mT); for magnetron sputtering: greater than about 1.3 Pa (10 mT); and for ion plating: greater than about 30 Pa (200 mT); and
- maintaining the angle of incidence of the coating flux on the surface to be coated at less than about 75°, and preferably less than about 30°

The metals used in the coating are those known to have an anti-microbial effect. For most medical devices, the metal must also be biocompatible. Preferred metals include the noble metals Ag, Au, Pt, Pd, and Ir as well as Sn, Cu, Sb, Bi, and Zn or alloys or compounds of these metals or other metals. Most preferred is Ag or Au, or alloys or compounds of one or more of these metals.

The coating is formed as a thin film on at least a part of the surface of the medical device. The film has a thickness no greater than that needed to provide release of metal ions on a sustainable basis over a suitable period of time. In that respect, the thickness will vary with the particular metal in the coating (which varies the solubility and abrasion resistance), and with the degree of atomic disorder in (and thus the solubility of) the coating. The thickness will be thin enough that the coating does not interfere with the dimensional tolerances or flexibility of the device for its intended utility. Typically, thicknesses of less than 1 micron have been found to provide sufficient sustained anti-microbial activity. Increased thicknesses may be used depending on the degree of metal ion release needed over a period of time. Thicknesses greater than 10 microns are more expensive to produce and normally should not be needed.

The anti-microbial effect of the coating is achieved when the device is brought into contact with an alcohol or a water based electrolyte such as, a body fluid or body tissue, thus releasing metal ions, atoms, molecules or clusters. The concentration of the metal which is needed to produce an anti-microbial effect will vary from metal to metal. Generally, anti-microbial effect is achieved in body fluids such as plasma, serum or urine at concentrations less than about 0.5 - 1.5 µg/ml.

The ability to achieve release of metal atoms, ions, molecules or clusters on a sustainable basis from a coating is dictated by a number of factors, including coating characteristics such as composition, structure, solubility and thickness, and the nature of the environment in which the device is used. As the level of atomic disorder is increased, the amount of metal ions released per unit time increases. For instance, a silver metal film deposited by magnetron sputtering at T/Tm <0.5 and a working gas pressure of about 0.9 Pa (7 mTorr) releases approximately 1/3 of the silver ions that a film deposited under similar conditions, but at 4 Pa (30 mTorr), will release over 10 days. Films that are created with an intermediate structure (ex. lower pressure, lower angle of incidence etc.) have Ag release values intermediate to these values as determined by bioassays. This then provides a method for producing controlled release metallic coatings in accordance with this invention. Slow release coatings are prepared such that the degree of disorder is low while fast release coatings are prepared such that the degree of disorder is high.

For continuous, uniform coatings, the time required for total dissolution will be a function of film thickness and the nature of the environment to which they are exposed. The relationship in respect of thickness is approximately linear, i.e. a two fold increase in film thickness will result in about a two fold increase in longevity.

It is also possible to control the metal release from a coating by forming a thin film coating with a modulated structure. For instance, a coating deposited by magnetron sputtering such that the working gas pressure was low (ex. 2 Pa (15 mTorr)) for 50% of the deposition time and high (ex. 4 Pa (30 mTorr)) for the remaining time, has a rapid initial release of metal ions, followed by a longer period of slow release. This type of coating is extremely effective on devices such as urinary catheters for which an initial rapid release is required to achieve immediate anti-microbial concentrations followed by a lower release rate to sustain the concentration of metal ions over a period of weeks.

The substrate temperature used during vapour deposition should not be so low that annealing or recrystallization of the coating takes place as the coating warms to ambient temperatures or the temperatures at which it is to be used (ex. body temperature). This allowable ΔT, that the temperature differential between the substrate temperature during deposition and the ultimate temperature of use, will vary from metal to metal. For the most preferred metals of Ag and Au, preferred substrate temperatures of -20 to 200°C, more preferably -10°C to 100°C are used.

Atomic disorder may also be achieved, in accordance with the present invention, by preparing composite metal materials, that is materials which contain one or more anti-microbial metals in a metal matrix which includes atoms or molecules different from the anti-microbial metals.

Our technique for preparing composite material is to co- or sequentially deposit the anti-microbial metal(s) with one or more other inert, biocompatible metals selected from Ta, Ti, Nb, Zn, V, Hf, Mo, Si, Al and alloys of these metals or other metal elements, typically other transition metals. Such inert metals have a different atomic radii from that of the anti-microbial metals, which results in atomic disorder during deposition. Alloys of this kind can also serve to reduce atomic diffusion and thus stabilize the disordered structure. Thin film deposition equipment with multiple targets for the placement of each of the anti-microbial and inert metals is preferably utilized. When layers are sequentially deposited the layer(s) of the inert metal(s) should be discontinuous, for example as islands within the anti-microbial metal matrix. The final ratio of the anti-microbial metal(s) to inert metal(s) should be greater than about 0.2. The most preferable inert metals are Ti, Ta, Zn and Nb. It is also possible to form the anti-microbial coating from oxides, carbides, nitrides, sulphides, borides, halides or hydrides of one or more of the and-microbial metals and/or one or more of the inert metals to achieve the desired atomic disorder.

Another composite material within the scope of the present invention is formed by reactively co- or sequentially depositing, by physical vapour techniques, a reacted material into the thin film of the anti-microbial metal(s). The reacted material is an oxide, nitride, carbide, boride, sulphide, hydride or halide of the anti-microbial and/or inert metal, formed in situ by injecting the appropriate reactants, or gases containing same, (ex. air, oxygen, water, nitrogen, hydrogen, boron, sulphur, halogens) into the deposition chamber. Atoms or molecules of these gases may also become absorbed or trapped in the metal film to create atomic disorder. The reactant may be continuously supplied during deposition for codeposition or it may be pulsed to provide for sequential deposition. The final ratio of anti-microbial metal(s) to reaction product should be greater than about 0.2. Air, oxygen, nitrogen and hydrogen are particularly preferred reactants.

The above deposition techniques to prepare composite coatings may be used with or without the conditions of lower substrate temperatures, high working gas pressures and low angles of incidence previously discussed. One or more of these conditions is preferred to retain and enhance the amount of atomic disorder created in the coating.

It may be advantageous, prior to depositing an anti-microbial in accordance with the present invention, to provide an adhesion layer on the device to be coated, as is known in the art. For instance, for a latex device, a layer of Ti, Ta or Nb may be first deposited to enhance adhesion of the subsequently deposited anti-microbial coating.

Anti-Microbial Powders

Anti-microbial powders, including nanocrystalline powders and powders made from rapidly solidified flakes or foils, can be formed with atomic disorder so as to enhance solubility. The powders either as pure metals, metal alloys or compounds such as metal oxides or metal salts, can be mechanically worked or compressed to impart atomic disorder. This mechanically imparted disorder is conducted under conditions of low temperature (i.e. temperatures less than the temperature of recrystallization of the material) to ensure that annealing or recrystallization does not take place. The temperature varies between metals and increases with alloy or impurity content.

Anti-microbial powders produced in accordance with this invention may be used in a variety of forms, for instance in topical creams, paints or adherent coatings. Alternatively, the powder may be incorporated into a polymeric, ceramic or metallic matrix to be used as a material for medical devices or coatings therefor.

Fine Grain or Nanocrystalline Materials of Anti-Microbial Metals

Methods of forming fine grain or nanocrystalline materials from the vapour phase are well known and documented in the literature. For instance, nanocrystalline materials may be formed by a modified standard inert-gas condensation technique. The material to be deposited is evaporated from an electrically heated boat or crucible into an inert gas atmosphere such as argon or helium with a pressure of about 5 to 7 Torr. The temperature of the boat has to be high enough to obtain a substantial vapour pressure of the material of interest. For metals, a temperature about 100°C above the melting point of the metal will typically provide an adequate vapour pressure. Due to interatomic collisions with the working gas atmosphere atoms, the evaporated atoms of the material lose their kinetic energy and condense onto a cold finger or substrate held at about 77 K (liquid nitrogen cooled) in the form of a lose powder or friable flakes or film, the grain size of which is less than about 20 nm. With respect to powders or flakes, a high vacuum (less than $5 \times 10^{-6}$ Pa) is restored and the powder or flakes are stripped off from the cold finger and collected in a cold trap.

Fine grain materials are produced analogously in gas condensation/vapour deposition processes, as is known in the art. This is typically achieved by altering the cold finger or substrate temperature and the gas pressure to allow the particle to coarsen to the desired size which is preferably under 5000 mm

Fine powders/nanocrystalline powders of anti-microbial metals prepared in accordance with the known prior art processes have been tested and found not to have sufficient anti-microbial efficacy. In order to introduce atomic disorder into the materials at a level which is sufficient to produce an anti-microbial effect, the anti-microbial metal, alloy or compound to be deposited is co-, sequentially or reactively deposited in a matrix with atoms or molecules of a different material (dopant) under conditions such that atomic disorder is created and retained in the matrix. The different material is selected from inert biocompatible metals, such as Ta, Ti, Nb, B, Hf, Zn, Mo, Si and Al, most preferably Ta, Ti and Nb. Alternatively the different material is an oxide, nitride, carbide, boride, sulphide or halide of either or both of an anti-microbial metal or of the biocompatible metal. A further alternative is to introduce the different material from the working gas atmosphere, either by reactive deposition or by absorbing or trapping atoms or molecules from the working gas into the matrix. Working gas atmospheres containing oxygen, nitrogen, hydrogen, boron, sulphur and halogens may be used. Working gas atmospheres including oxygen are most preferred, such that the matrix of anti-microbial metal includes either or both of trapped oxygen and oxides of the anti-microbial metal.

A further technique for forming anti-microbial powders of the present invention is to form coatings containing atomic disorder in the manner set out above onto an inert, preferably biocompatible, particulate material such as talc, bentonite, cornstarch or ceramics such as alumina. The particles may be coated by physical vapour deposition techniques under conditions to create atomic disorder, as set forth above in respect of the anti-microbial metal coatings. Alternatively, the powders can be coated by adapting a vapour deposition process, for instance by passing a vapour of the anti-microbial material through a fixed porous bed of the powders, by fluidizing the powder bed in the anti-microbial metal vapour phase, or by letting the powder fall through a vapour of the anti-microbial material. In all cases, the powder could be cooled and/or the working gas atmosphere could be altered to include a different material (ex. oxygen), in order to produce the desired degree of atomic disorder.

Activation of Anti-Microbial Materials

Irradiation of anti-microbial materials (powders, nanocrystalline powders, foils, coatings or composite coatings of anti-microbial metals) which contain atomic disorder formed by any of the above-described procedures, will further

9

activate or enhance the anti-microbial effect Thus, even materials having a low level of atomic disorder may be activated to an anti-microbial level.

Irradiation is performed with any low linear energy transfer form of radiation, including beta, gamma and x-rays. Gamma radiation at a dose of 1 Mrad or greater is preferred. Since gamma radiation is an acceptable method of sterilization of medical devices, activation and sterilization may be achieved simultaneously through the irradiation process of the present invention.

The irradiation step is preferably conducted such that the anti-microbial material being irradiated is oriented generally perpendicular to the incoming radiation (rather than parallel). A further enhancement of the anti-microbial effect can be achieved by conducting the irradiation step with a dielectric material adjacent to, or preferably sandwiched around the anti-microbial material. Exemplary dielectrics include oxides of Si, Ti, Ta and Al. Silicon oxide surfaces are preferred. It is believed that the dielectric material provides forward scattering of electrons into the anti-microbial coating.

Without being bound by the same it is believed that the irradiation step is causing one or more of the following changes in the anti-microbial material:

1) creating further atomic disorder, such as point defects;

2) enhancing oxygen adsorption/chemisorption to the surface of the anti-microbial material;

3) activating trapped dopant atoms or molecules such as oxygen to $O^+$ or $O_2^-$; and

4) creating broken or dangling bonds at the surface.

With respect to the second and third proposed mechanisms, it is possible that oxygen adsorption/chemisorption and/or activation creates a super saturated concentration of $O_2$, $O^+$ or $O_2^-$ species in or on the anti-microbial metal surface, which results in the more rapid dissolution of the anti-microbial metal or species thereof into an aqueous environment through the generation of various chemical species of the anti-microbial metal, including oxides and hydroxides.

Silver Materials Forming Complex Silver Ions

In accordance with the invention, silver materials are prepared which form complex silver ions other than $Ag^+$, $Ag^{2+}$ and $Ag^{3+}$, when the material is contacted with an alcohol or a water based electrolyte. Exemplary complex silver ions shown to demonstrate an anti-microbial effect include $Ag(CN)_2^-$, $AgCN_{(aq)}$(ion pair), $Ag(NH_3)_2^+$, $AgCl_2^-$, $Ag(OH)_2^-$, $Ag_2(OH)_3^-$, $Ag_3(OH)_4^-$ and $Ag(S_2O_3)_2^{3-}$. These silver materials forming complex silver ions have wide application, for instance, as anti-microbial coatings for medical devices, as anti-microbial powders for medical or pharmaceutical use, as anti-fouling paints, coatings or compositions, anti-microbial coatings for filters and the like.

It should be understood that the phrase "silver materials which form complex silver ions other than $Ag^+$, $Ag^{2+}$ and $Ag^{3+}$" as used herein and in the claims is not intended to exclude silver materials which form one or more of $Ag^+$, $Ag^{2+}$ and $Ag^{3+}$, ions in addition to the complex silver ions when the material contacts an alcohol or a water based electrolyte. The notation $Ag^+$, $Ag^{2+}$ and $Ag^{3+}$ refers to these ions in solution and includes solvated forms. The term complex silver ions as used herein and in the claims is not intended to include silver ions stabilized with strong oxidizing agents, such as persulphate and periodate, to prevent the reduction of the silver ions.

The anti-microbial coatings, powders and foils of the present invention, when created with atomic disorder as above described, are exemplary of silver materials which form complex silver ions other than $Ag^+$ so as to cause an anti-microbial effect. It is believed that the complex silver ions which may be formed when such silver materials contact an alcohol or water based electrolyte, are one or more of the negative ions $Ag(OH)_2^-$, $Ag_2(OH)_3^-$ and $Ag_3(OH)_4^-$.

Silver materials which form complex silver ions may also be prepared by bringing a silver metal, compound or salt into an environment containing excessive amounts of a cationic, anionic or neutral species with which it is desired to complex silver. For example, the negative complex silver ion $AgCl_2^-$ can be generated by placing a silver salt such as $AgNO_3$ in an aqueous medium with an elevated concentration of the $Cl^-$ ion. $AgNO_3/NaCl$ or $AgCl/NaCl$ mixtures, solutions or suspensions can form the $AgCl_2^-$ ion. This $AgCl_2^-$ ion may also be generated with mixtures of silver powder with NaCl. Preferably the silver powder is one which is prepared in accordance with the present invention so as to contain atomic disorder, but bulk silver may also be activated in this manner. Bulk silver powder, fine grain (<140 nm) and nanocrystalline (<20 nm) powders may be used. Similarly, the ion $Ag(NH_3)_2^+$ can be formed in aqueous solution by adding silver salts to excess ammonium hydroxide. The ion $Ag(S_2O_3)_2^{3-}$ may be formed in aqueous solution by adding silver salts to excess sodium thiosulphate. The ion $Ag(CN)_2^-$ may be formed in aqueous solution by adding excess potassium cyanide to silver cyanide.

The silver materials forming complex silver ions may be formulated for use in many forms, including for example, powders, suspensions, solutions, ointments or coatings. For instance, a pharmaceutical composition to generate the $AgCl_2^-$ ion can be formulated as a mixture of the salts $AgNO_3/NaCl$ or as a mixture of NaCl with a silver powder, preferably one containing atomic disorder. These mixtures of the silver material might be pre-formulated as a solution, suspension or ointment with a sterile aqueous or saline solution and pharmaceutically acceptable carriers, diluents, exipients and the like. Alternatively the silver material might be provided as the mixtures of silver powder/NaCl salt or $AgNO_3/NaCl$, for later formulation by the end user.

Physical/Chemical Characteristics of Anti-Microbial Silver Material

The modified metal materials formed in accordance with the present invention so as to contain atomic disorder which leads to enhanced release of the metal species have novel physical characteristics when compared with materials in their normal ordered crystalline state. Silver materials made in accordance with the present invention have been characterized as having the following novel characteristics:

- a positive rest potential, $E_{rest}$ for example, when measured against an SCE reference electrode in a 1 M KOH solution;
- preferably a ratio of temperature of recrystallization to melting temperature less than 0.33, and most preferably less than 0.30;
- preferably a temperature of recrystallization less than about 140°C; and
- preferably a grain size less than about 200nm, more preferably less than 140nm and most preferably less than 90nm.

Analysis of the silver materials by XRD, XPS and SIMS techniques confirms the chemical nature and content of the film as silver metal, and in the event that the material is formed with oxygen in the working gas atmosphere, one or both of silver oxide and trapped oxygen. TEM analysis reveals growth twins in the silver material, which are converted to annealed twins when the materials are annealed above the temperature of recrystallization.

The invention is further illustrated by the following non-limiting examples.

Example 1

A medical suture material size 2/0, polyester braid was coated by magnetron sputtering 20.3 cm diameter (8 in.) from planar silver and copper magnetron cathodes to form an Ag-Cu-alloy on the surface to a thickness of 0.45 microns, using either argon gas working pressures of 0.9 Pa (7 mTorr) or 4 Pa (30 mT) at 0.5 KW power and a T/Tm ratio of less than 0.5. The total mass flow of gas was 700 sccm (standard cubic centimeters per minute).

The anti-microbial effect of the coatings was tested by a zone of inhibition test. Basal medium Eagle (BME) with Earle's salts and L-glutamine was modified with calf/serum (10%) and 1.5 % agar prior to being dispensed (15 ml) into Petri dishes. The agar containing Petri plates were allowed to surface dry prior to being inoculated with a lawn of *Staphylococcus aureus* ATCC# 25923. The inoculant was prepared from Bactrol Discs (Difco, M.) which were reconstituted as per the manufacturer's directions. Immediately after inoculation, the materials or coatings to be tested were placed on the surface of the agar. The dishes were incubated for 24 h at 37°C. After this incubation period, the zone of inhibition was measured and a corrected zone of inhibition was calculated (corrected zone of inhibition = zone of inhibition - diameter of the test material in contact with the agar ).

The results showed no zone of inhibition on the uncoated suture, a zone of less than 0.5 mm around the suture coated at 0.9 Pa (7 mTorr) and a zone of 13 mm around the suture coated at 4 Pa (30 mTorr). Clearly the suture coated in accordance with the present invention exhibits a much more pronounced and effective anti-microbial effect.

Example 2

This example is included to illustrate the surface structures which are obtained when silver metal is deposited on silicon wafers using a magnetron sputtering facility and different working gas pressures and angles of incidence (i.e. the angle between the path of the sputtered atoms and the substrate). All other conditions were as follows: target was a 20.3 cm diameter planar silver magnetron cathode; power was 0.1 kW; deposition rate was 200 A°/min; ratio of temperature of substrate (wafer) to melting point of silver (1234°K), T/Tm was less than 0.3. Argon gas pressures of 0.9 Pa (7 mTorr) (a normal working pressure for metal coatings) and 4 Pa (30 mTorr) were used with a total mass flow of 700 sccm. Angles of incidence at each of these pressures were 90° (normal incidence), 50° and 10°. The coatings had a thickness of about 0.5 microns.

The resulting surfaces were viewed by scanning electron microscope. As argon gas pressure increased from 0.9 Pa (7 mTorr) to 4 Pa (30 mTorr) the grain size decreased and void volume increased significantly. When the angle of incidence was decreased, the grain size decreased and the grain boundaries became more distinct. At 0.9 Pa (7 mTorr) argon pressure and an angle of incidence of 10°, there were indications of some voids between the grains. The angle of incidence had a greater effect on the surface topography when the gas pressure was increased to 4 Pa (30 mTorr). At 90°, the grain size varied from 60 - 150 nm and many of the grains were separated by intergrain void spaces which were 15 - 30 nm wide. When the angle of incidence was decreased to 50°, the grain size decreased to 30 - 90 nm and the void volume increased substantially. At 10°, the grain size was reduced to about 10 - 60 nm and void volumes were increased again.

The observed nanometre scale changes in surface morphology and topography are indications of atomic disorder in the silver metal. While not being bound by the same, it is believed that such atomic disorder results in an increase in the chemical activity due to increased internal stresses and surface roughness created by mismatched atoms. It is believed that the increased chemical activity is responsible for the increased level of solubility of the coatings when in contact with an electrolyte such as body fluid.

The anti-microbial effect of the coatings was evaluated using the zone of inhibition test as set out in Example 1. Each coated silicon wafer was placed on an individual plate. The results were compared to the zones of inhibition achieved when solid silver (i.e. greater than 99% silver) sheets, wires or membranes were tested. The results are summarized in Table 1. It is evident that the pure silver devices and the silver sputtered coating at 0.9 Pa (7 mTorr) do not produce any biological effect. However, the coatings deposited at a higher than normal working gas pressure, 4 Pa (30 mTorr), demonstrated an anti-microbial effect, as denoted by the substantial zones of inhibition around the discs. Decreasing the angle of incidence had the greatest effect on anti-microbial activity when combined with the higher gas pressures.

Table I

| Anti-microbial effects of various silver and silver coated samples as determined using ***Staphylococcus aureus*** | | | | |
|---|---|---|---|---|
| Sample | Percent Silver | Angle of Deposition | Working Gas Pressure Pa (mTorr) | Corrected Zone of Inhibition (mm) |
| Silver Sheet-rolled | 99+ | - | - | <0.5 |
| Silver wire (.0045") | 99+ | - | - | <0.5 |
| Silver membrane-cast | 99+ | - | - | <0.5 |
| Sputtered thin film | 99+ | normal (90°) | 0.9(7) | <0.5 |
| Sputtered thin film | 99+ | 50° | 0.9(7) | <0.5 |
| Sputtered thin film | 99+ | 10° | 0.9(7) | <0.5 |
| Sputtered thin film | 99+ | normal (90°) | 4(30) | 6.3 |
| Sputtered thin film | 99+ | 50° | 4 (30) | 10 |
| Sputtered thin film | 99+ | 10 | 4 (30) | 10 |

Example 3

Silicon wafers were coated by magnetron sputtering using 20.3 cm diameter planar silver and copper magnetron cathodes to produce an alloy of Ag and Cu (80:20) at normal incidence at working gas pressures of 0.9 Pa (7 mTorr) and 4 Pa (30 mTorr), all other conditions being identical to those set out in Example 2. As in Example 2, when the coatings were viewed by SEM, the coatings formed at high working gas pressure had smaller grain sizes and larger void volumes than did the coatings formed at the lower working gas pressures.

Coatings which were similarly formed as a 50:50 Ag/Cu alloy were tested for anti-microbial activity with the zone of inhibition test set out in Example 1. The results are summarized in Table 2. Coatings deposited at low working gas pressure (0.9 Pa (7 mTorr)) showed minimal zones of inhibition, while the coatings deposited at high working gas pressure (4 Pa (30 mTorr)) produced larger zones of inhibition, indicative of anti-microbial activity.

Table 2

| The anti-microbial effect of various sputter deposited silver-copper alloys as determined using *Staphylococcus aureus* | | | | |
|---|---|---|---|---|
| Sample | Percent Silver | Angle of Deposition (°) | Working Gas Pressure Pa (mTorr) | Corrected Zone of Inhibition (mm) |
| 1 | 50 | normal (90°) | 1.0 (7.5) | <0.5 |
| 2 | 50 | normal (90°) | 4 (30) | 16 |
| 3 | 50 | 10 | 4 (30) | 19 |

Example 4

A coating in accordance with the present invention was tested to determine the concentration of silver ions released into solution over time. One $cm^2$ silicon wafer discs were coated with silver as set forth in Example 2 at 0.9 Pa (7 mTorr) and 4 Pa (30 mTorr) and normal incidence to a thickness of 5000 A°. Using the method of Nickel et al., Eur. J. Clin. Microbiol., 4(2), 213-218, 1985, a sterile synthetic urine was prepared and dispensed into test tubes (3.5 ml). The coated discs were placed into each test tube and incubated for various times at 37°C. After various periods of time, the discs were removed and the Ag content of the filtered synthetic urine was determined using neutron activation analysis.

The results are set forth in Table 3. The table shows the comparative amounts of Ag released over time from coatings deposited on discs at 0.9 Pa (7 mTorr) or 4 Pa (30 mTorr). The coatings deposited at high pressure were more soluble than those deposited at low pressure. It should be noted that this test is a static test. Thus, silver levels build up over time, which would not be the case in body fluid where there is constant turn over.

Table 3

| Concentration of silver in synthetic urine as a function of exposure time Silver Concentration µg/ml | | |
|---|---|---|
| Exposure Time (Days) | Working Argon gas pressure 0.9 Pa (7mTorr) | Working argon gas pressure 4 Pa (30mTorr) |
| 0 | NDI | ND |
| 1 | 0.89 | 1.94 |
| 3 | 1.89 | 2.36 |
| 10 | 8.14 | 23.06 |
| Note: Films were deposited at normal incidence (90°) 1 - ND (non detectable) <0.46 µg/ml | | |

Example 5

This example is included to illustrate coatings in accordance with the present invention formed from another noble metal, Pd. The coatings were formed on silicon wafers as set forth in Example 2, to a thickness of 5000 A°, using 0.9 Pa (7 mTorr) or 4 Pa (30 mTorr) working gas pressures and angles of incidence of 90° and 10°. The coated discs were evaluated for anti-microbial activity by the zone of inhibition test substantially as set forth in Example 1. The coated discs were placed coating side up such that the agar formed a 1 mm surface coating over the discs. The medium was allowed to solidify and surface dry, after which the bacterial lawn was spread over the surface. The dishes were incubated at 37°C for 24 h. The amount of growth was then visually analyzed.

The results are set forth in Table 4. At high working gas pressures, the biological activity of the coating was much greater than that of coatings deposited at low pressure. Changing the angle of incidence (decreasing) improved the anti-microbial effect of the coating to a greater extent when the gas pressure was low than when it was high.

Table 4

| Surface Control of **Staphylococcus aureus** by Sputter Deposited Palladium metal | | | |
|---|---|---|---|
| Sample | Sputtering Pressure Pa (mTorr) | Angle of Deposition | Anti-microbial Control |
| 1 | 0.9 (7) | 90°(normal incidence) | More than 90% of surface covered by bacterial growth |
| 2 | 0.9 (7) | 10°(grazing incidence) | 20-40% of surface covered by bacterial growth |
| 3 | 4 (30) | 90°(normal incidence) | Less than 10% surface covered by bacterial growth |

Example 6

This example is included to illustrate the effect of silver deposition temperature on the anti-microbial activity of the coating. Silver metal was deposited on 2.5 cm sections of a latex Foley catheter using a magnetron sputtering facility. Operating conditions were as follows; the deposition rate was 200 A° per minute; the power was 0.1 kW; the target was a 20.3 cm diameter planar silver magnetron cathode; the argon working gas pressure was 4 Pa (30mTorr); the total mass flow was 700 sccm; and the ratio of temperature of substrate to melting point of the coating metal silver, $T/T_m$ was 0.30 or 0.38. In this example the angles of incidence were variable since the substrate was round and rough. That is the angles of incidence varied around the circumference and, on a finer scale, across the sides and tops of the numerous surface features. The anti-microbial effect was tested by a zone of inhibition test as outlined in Example 1.

The results showed corrected zones of inhibition of 0.5 and 16 mm around the tubing coated at $T/T_m$ values of 0.38 and 0.30 respectively. The sections of Foley catheter coated at the lower $T/T_m$ value were more efficacious than those coated at higher $T/T_m$ value.

Example 7

This example is included to demonstrate an anti-microbial coating formed by DC magnetron sputtering on a commercial catheter. A teflon coated latex Foley catheter was coated by DC magnetron sputtering 99.99% pure silver on the surface using the conditions listed in Table 5. The working gases used were commercial Ar and 99/1 wt% Ar/O$_2$.

The anti-microbial effect of the coating was tested by a zone of inhibition test. Mueller Hinton agar was dispensed into Petri dishes. The agar plates were allowed to surface dry prior to being inoculated with a lawn of *Staphylococcus aureus* ATCC# 25923. The inoculant was prepared from Bactrol Discs (Difco, M.) which were reconstituted as per the manufacturer's directions. Immediately after inoculation, the coated materials to be tested were placed on the surface of the agar. The dishes were incubated for 24 hr. at 37°C. After this incubation period, the zone of inhibition was measured and a corrected zone of inhibition was calculated (corrected zone of inhibition = zone of inhibition - diameter of the test material in contact with the agar ).

The results showed no zone of inhibition for the uncoated samples and a corrected zone of less than 1 mm for catheters sputtered in commercial argon at a working gas pressure of 0.7 Pa (5 mT). A corrected zone of inhibition of 11 mm was reported for the catheters sputtered in the 99/1 wt% Ar/O$_2$ using a working gas pressure of 5.3 Pa (40 mT). XRD analysis showed that the coating sputtered in 1% oxygen was a crystalline Ag film. This structure clearly caused an improved anti-microbial effect for the coated catheters.

Table 5

| Conditions of DC Magnetron Sputtering Used for Anti-Microbial Coatings | |
|---|---|
| Samples Sputtered in Commercial Argon | Samples Sputtered in 99/1 wt% Ar/O$_2$ |
| Power 0.1 kW | Power 0.5 kW |
| Target 20.3 cm dia Ag | Target 20.3 cm dia Ag |
| Argon Pressure: 0.7 Pa (5 m Torr) | Ar/O$_2$ Pressure: 5.3 Pa (40 m Torr) |
| Total Mass Flow: 700 sccm | Total Mass Flow: 700sccm |

Table 5 (continued)

| Conditions of DC Magnetron Sputtering Used for Anti-Microbial Coatings | |
| --- | --- |
| Samples Sputtered in Commercial Argon | Samples Sputtered in 99/1 wt% Ar/O$_2$ |
| Initial Substrate Temperature: 20°C | Initial Substrate Temperature: 20°C |
| Cathode/Anode Distance: 40 mm | Cathode/Anode Distance: 100mm |
| Film Thickness: 2500 Å | Film Thickness: 3000 Å |

Example 8

This example demonstrates silver coatings formed by arc evaporation, gas scattering evaporation (pressure plating) and reactive arc evaporation. Evaporation of 99.99% silver was performed onto silicon or alumina wafers at an initial substrate temperature of about 21°C, using the parameters as follows:

Bias: -100 V

Current: 20 Amp-hrs

Angle of incidence: 90°

Working Gas Pressure: 0.001 Pa (0.01 mT) (arc), 3.5 Pa (26 mT) Ar/H$_2$ 96:4 (gas scattering evaporation), and 3.5 Pa (26 mT) O$_2$ (reactive arc evaporation)

No corrected ZOI was observed for wafers coated at vacuum (arc). Pressure plating with a working gas atmosphere containing Ar and 4 % hydrogen produced a 6 mm ZOI, while a working gas atmosphere of pure oxygen (reactive arc) produced an 8 mm ZOI. Film thicknesses of about 4000 Angstroms were produced. The results indicate that the presence of gases such as hydrogen and/or oxygen in the arc evaporation atmosphere cause the coatings to have improved anti-microbial efficacy.

Example 9

This example is included to illustrate composite materials to produce anti-microbial effects. A set of coatings were produced by RF magnetron sputtering zinc oxide onto silicon wafers as outlined below. The zinc oxide coatings showed no zone of inhibition.
Coatings of Ag and ZnO were deposited to a total thickness of 3300 Angstroms by sequentially sputtering layers of Ag with layers of ZnO, according to the conditions below, in a 75/25 wt% ratio. The coatings were demonstrated to have no zone of inhibition when the zinc oxide layers were about 100 Angstroms thick. However, films consisting of islands of very thin to discontinuous layers of ZnO (less than 50 Angstroms) in an Ag matrix (ie. a composite film) had an 8 mm corrected zone of inhibition.
The conditions used to deposit ZnO were as follows:

Target 203 cm dia ZnO; Working gas = argon; Working gas pressure = 4 Pa (30 mT); Cathode-Anode distance: 40 mm; Initial Substrate Temperature: 21°C; Power RF magnetron. 0.5 kW.

The conditions used to deposit the Ag were as follows:

Target 20.3 cm dia Ag; Working gas =argon; Working gas pressure = 4 Pa (30 mT); Cathode-Anode distance = 40 mm; Initial Substrate Temperature = 21°C; Power = DC magnetron, 0.1 kW.

Example 10

This example demonstrates the effects of cold working and annealing silver and gold powders on the anti-microbial efficacy demonstrated by a standard zone of inhibition test. Cold working of such powders results in a defective surface structure containing atomic disorder which favours the release of ions causing anti-microbial activity. The anti-microbial effect of this defective structure can be removed by annealing.

Nanocrystalline silver powder (crystal size about 30 nm) was sprinkled onto adhesive tape and tested. A zone of inhibition of 5 mm was obtained, using the method set forth in Example 7. A 0.3g pellet of the nanocrystalline Ag powder was pressed at 275,700 kpa (kiloPascal) (40,000 psi). The pellet produced a 9 mm zone of inhibition when tested for anti-microbial activity. Nanocrystalline silver powder was mechanically worked in a ball mill for 30 sec. The resulting powder was tested for anti-microbial activity, both by wrinkling the worked powder on adhesive tape and applying to the plates, and by pressing the powder into a pellet at the above conditions and placing the pellet on the plates. The zones of inhibition observed were 7 and 11 mm respectively. A pellet that had been pressed from the worked powder was annealed at 500°C for 1 hour under vacuum conditions. A reduced zone of inhibition of 3 mm was observed for the annealed pellet.

These results demonstrate that nanocrystalline silver powder, while having a small anti-microbial effect on its own, has an improved anti-microbial effect by introducing atomic disorder by mechanical working of the powder in a ball mill or by pressing it into a pellet. The anti-microbial effect was significantly decreased by annealing at 500°C. Thus, conditions of mechanical working should not include or be followed by conditions such as high temperature, which allow diffusion. Cold mechanical working conditions are preferred to limit diffusion, for example by working at room temperature or by grinding or milling in liquid nitrogen.

Silver powder, 1 micron particle size, was tested in a manner similar to above. The Ag powder was sprinkled onto adhesive tape and tested for a zone of inhibition. No zone of inhibition was observed. The powder was worked in a ball mill for 30 seconds and sprinkled onto adhesive tape. A 6 mm zone of inhibition was observed around the powder on the tape. When the Ag powder(as is or after mechanical working in the ball mill) was pressed into a 0.3 g pellet using 275,700 kPa (40,000 psi), zones of inhibition of 5 and 6 mm respectively were observed. A pellet which was formed from the ball milled powder and which was annealed at 500°C for 1 hour had significantly reduced anti-microbial activity. Initially the pellet had some activity (4.5 mm zone of inhibition) but after the pellet was tested a second time, no zone of inhibition was observed. A control pellet which had not been annealed continued to give a zone of inhibition greater than 4 mm even after 14 repeats of the test. This demonstrates that an annealing step, following mechanical working, limits the sustainable release of the anti-microbial silver species from the powders.

Nanocrystalline gold (20 nm crystals), supplied as a powder, was tested for anti-microbial effect by sprinkling the powder onto adhesive tape and using the zone of inhibition test. No zone of inhibition was recorded for the nanocrystalline gold powder. The gold powder was pressed into a 0.2 g pellet using 275,700 kPa (40,000 psi). A 10 mm zone of inhibition was observed. When the pressed pellets were subsequently vacuum annealed at 500°C for 1 hour and the zone of inhibition was found to be 0 mm.

The results showed that solubility and thus the anti-microbial efficacy of gold powders can be improved by a mechanical working process such as pressing a nanocrystalline material into a pellet. The anti-microbial activity can be removed by annealing. Cold working is preferred.

Other gold powders including a 2-5 micron and a 250 micron particle size powder did not demonstrate an anti-microbial effect under the above mechanical working conditions. It is believed that the small grain size of the nanocrystalline gold powder was an important cofactor which, with the mechanical working, produced the desired anti-microbial effect.

Example 11

This example is included to demonstrate a composite anti-microbial coating formed by reactive sputtering (another example of composite films). Example 7 demonstrates that an anti-microbial coating of silver can be obtained by sputtering in argon and 1% oxygen (0.5 kW, 5.3 Pa (40 mTorr), 100 mm anode/cathode distance, and 20°C - produced a zone of inhibition of 11 mm).

When a working gas of argon and 20 wt% oxygen was used to sputter anti-microbial coatings under the conditions listed below, the zones of inhibition ranged from 6 to 12 mm. This indicates that the provision of a reactive atmosphere during vapour deposition has the result of producing an anti-microbial film over a wide range of deposition process parameters.

Table 6

| Sputtering Conditions | |
|---|---|
| Target | 20.3 cm dia, 99.99% Ag |
| Working Gas: | 80/20 wt% $Ar/O_2$ |
| Working Gas Pressure: | 0.3 to 6.7 Pa (2.5 to 50 mTorr) |

Table 6 (continued)

| Sputtering Conditions | |
|---|---|
| Total Mass Gas Flow: | 700 sccm |
| Power: | 0.1 to 2.5 kW |
| Substrate Temperature: | -5 to 20°C |
| Anode/Cathode Distance | 40 to 100 mm |
| Base Pressure: | less than 5 x $10^{-4}$ Pa (4 x $10^{-6}$ Torr) |

Example 12

This example demonstrates that the coatings of this invention have an anti-microbial effect against a broad spectrum of bacteria.

A total of 171 different bacterial samples encompassing 18 genera and 55 species were provide by the Provincial Laboratory of Public Health for Northern Alberta. These samples had been quick frozen in 20% skim milk and stored at -70°C for periods ranging from several months to several years. Fastidious organisms which were unlikely to grow under conditions used in standard Kirby-Bauer susceptibility testing were not used.

Each frozen sample was scraped with a sterile cotton swab to inoculate a blood agar plate (BAP). The plates were incubated overnight at 35°C. The following morning isolated colonies were subcultured onto fresh BAPs and incubated at 35°C overnight. The next day, the organisms were subjected to Kirby-Bauer susceptibility testing as described below.

Four to five colonies (more if colonies were small) of the same morphological type were selected from each BAP subculture and inoculated into individual tubes containing approximately 5 mL of tryptic soy broth (TSB). The broths were incubated at 35°C for approximately 2 to 3 hours. At this time, the turbidity of most of the broth cultures either equalled or exceeded that of a 0.5 McFarland standard. The more turbid samples were diluted with sterile saline to obtain a turbidity visually comparable to that of the standard. To aid in the visual assessment of turbidity, tubes were read against a white background with contrasting black line.

A small number of the organisms (*Streptococcus* and *Corynebacterium*) did not grow well in TSB. The turbidity of these broths, after incubation, was less than that of the 0.5 McFarland standard. Additional colonies from the BAP subcultures were inoculated to these tubes to increase the turbidity to approximate that of the standard.

Within 15 minutes of adjusting the turbidity of the bacterial suspensions a sterile cotton swab was dipped into each broth. Excess fluid was removed by rotating the swab against the rim of the tube. The inoculum was applied to a Mueller Hinton (MH) agar plate by streaking the swab evenly in three directions over the entire agar surface. Three 1 cm x 1 cm silver coated silica wafer squares were applied to each MH plate and the plates were inverted and incubated overnight at 35°C. The coatings had been sputtered under the following conditions, which through XRD analysis were shown to be silver/silver oxide composite films:

| Target: | 20.3 cm dia, 99.99% Ag |
|---|---|
| Working gas: | 80/20 wt % Ar/$O_2$ |
| Working gas pressure: | 5.3 Pa (40 mT) |
| Total Mass Gas Flow: | 700 sccm |
| Power: | 0.1 kW |
| Temperature of Deposition | 20°C |
| Base pressure | 2.7 X $10^{-4}$ Pa (2 x $10^{-6}$ Torr) |
| Cathode/anode distance | 40 mm |

BAP cultures of control organisms were provided by the Provincial Laboratory and included: *Staphylococcus aureus* ATCC 25923; *Pseudomonas aeruginosa* ATCC 27853; *Escherichia coli*: ATCC 25922; and *Enterococcus faecalis* ATCC 29212 to check the quality of the MH agar. These cultures were treated in a like manner to the test organisms except that standard antibiotic discs rather than silver coated wafers were applied to the bacterial lawns on the MH agar. These organisms demonstrated that the MH agar was suitable for standard ZOI tests.

After 16 to 18 hours of incubation at 35°C zones of inhibition around the silver wafers or antibiotic discs were measured to the nearest mm. Corrected zones were calculated by subtracting the size of the wafer (1 cm) from the size of the total zone. Representative zone of inhibition results are shown in Table 7.

Table 7

| The Sensitivity of a Broad Range of Microorganisms to Silver* Coated Silicon Wafers | | |
|---|---|---|
| Organism | Source | Corrected Zone of Inhibition (mm) |
| *Staphylococcus epidermidis* RC-455 | blood | 10 |
| *Bacillus licheniformis* R-2138 | tibia | 6 |
| *Corynebacterium sp* R-594 | leg | 10 |
| *Listeria monocytogenes* R-590 | blood | 5 |
| *Enterococcus faecalis* SR-113 | bone | 5 |
| *Streptococcus bovis* SR-62 | blood | 10 |
| *Escherichia coli* R-1878 | urine | 11 |
| *Klebsiella ozonae* R-308/90 | abdomen | 10 |
| *Enterobacter cloacae* R-1682 | unknown | 8 |
| *Proteus vulgaris* 3781 | urine | 4 |
| *Providencia stuartii* U-3179 | urine | 8 |
| *Citrobacter freundii* U-3122/90 | urine | 7 |
| *Salmonella typhimirium* ER-1154 | urine | 6 |
| *Serraria marcescens* R-850 | sputum | 6 |
| *Pseudomonas aeruginosa* U-3027 | urine | 10 |
| *Xanthomonas maltophila* 90-10B | unknown | 9 |
| *Aeromonas caviae* R-1211 | wound | 5 |
| *Branhamella catarrhalis* R-2681 | unknown | 12 |

Silver deposition*

## Example 13

This example demonstrates the use of tantalum as an adhesive layer for coatings of this invention. Tantalum is well known as a material which, in the form of an interlayer, improves adhesion of thin films to substrates. In this example test sections including a group of stainless steel (316) (1 x 1 cm) and silicon (1.7 X 0.9 cm) coupons and sections of latex tubing (5 cm) were cleaned in ethanol and then half of the test sections were coated (by sputtering) with a thin layer (approx. 100 Angstroms) of Ta before an anti-microbial silver film was deposited on them. The second group of the test sections were only coated with the anti-microbial Ag film. Coating conditions are listed below. While all test sections had similar anti-microbial activity, the Ta coated test sections had much better adhesion properties than did the untreated test sections. Adhesion properties were determined using ASTM method D3359-87, a standard test method for measuring adhesion.

| Sputtering Conditions | |
|---|---|
| Target: | 20.3 cm dia, 99.99% Ta |

(continued)

| Sputtering Conditions | |
|---|---|
| Working Gas: | 99/1 wt% Ar/$O_2$ |
| Working Gas Pressure: | 1.3 Pa (10 mTorr) |
| Total Mass Gas Flow: | 700 sccm |
| Power: | 0.5 kW |
| Cathode/Anode Distance: | 100 mm |
| Substrate Temperature: | 20°C |
| | |
| Target: | 20.3 cm dia, 99.99% Ag |
| Working Gas: | 99/1 wt% Ar/$O_2$ |
| Working Gas Pressure: | 5.3 Pa (40 mTorr) |
| Total Mass Gas Flow: | 700 sccm |
| Power: | 0.5 kW |
| Cathode/Anode Distance: | 100 mm |
| Substrate Temperature: | 20°C |

Example 14

DC magnetron sputtering was used to deposit silver from a 20.3 cm dia, 99.98% pure cathode onto silicon and alumina wafers with commercial argon moisturized with water as the working gas at a total mass gas flow of 700 sccm. The argon was moisturized by passing it through two flasks containing 3 litres of room temperature water and one empty flask set up with glass wool to absorb any free liquid before the gas entered the sputtering unit.

The conditions of sputtering and the results of the standard zone of inhibition test performed on the sputtered silver films are shown below. Silver films which normally bad no anti-microbial properties when deposited using argon that had not been created with water yielded a corrected zone of inhibition of up to 8 mm when sputtered using an argon/water vapour mixture as the working gas.

Table 8

| Conditions used for DC Magnetron Sputtering of Anti-Microbial Coatings | | | | | |
|---|---|---|---|---|---|
| Working Gas | Working Gas Pressure Pa(mTorr) | Power | Substrate Temperature | Anode/Cathode Distance | Corrected Z0I |
| Commercial Argon | 1.3(10) | 0.5kW | -10°C | 100 mm | 0 mm |
| Ar passed through $H_2O$ | 1.3(10) | 0.5kW | -10°C | 100 mm | 8 mm |

Example 15

This example is included to illustrate the method of activating coatings with radiation, in accordance with another aspect of the present invention.

A series of 1.9 x 0.7 cm silicon wafers were coated with 3000 Å coatings of silver metal using DC magnetron sputtering under the following conditions:

| Sputtering Conditions | |
|---|---|
| Target | 20.3 cm dia, 99.99% Ag |

(continued)

| Sputtering Conditions | |
| --- | --- |
| Working Gas | 99/1 wt% Ar/O$_2$ |
| Working Gas pressure | 5.3 Pa (40 mTorr) |
| Total Mass Gas Flow | 700 sccm |
| Power | 0.5kW |
| Substrate Temperature | 21°C |
| Anode/Cathode Distance | 100 mm |

The coated wafers were divided into 4 groups and irradiated with varying doses of gamma radiation -0,1,2 and 4 megarad doses - from a $^{60}$Co source at Isomedix Inc., Morton Grove, Il., U.S.A. The samples were placed generally perpendicular to the incoming radiation. After irradiation, the samples were tested for biological activity (anti-microbial effect) using a standard zone of inhibition test on Mueller Hinton Agar (Difco, Mi) with *S. aureus* (ATCC#25923), as set out in previous examples. The results are summarized in Table 9.

Table 9

| Effects of Gamma Radiation on Biological Activity of Anti-Microbial Coatings | |
| --- | --- |
| Gamma Radiation Dose (megarads) | Corrected Zone of Inhibition (mm) |
| 0 | 11 |
| 1 | 14 |
| 2 | 17 |
| 4 | 20 |

The results generally show a log dose response relationship between the radiation dose and the observed biological response to the wafers. This illustrates that the gamma radiation has further activated the coatings of the present invention to enhance the anti-microbial effect.

The experiment was repeated with the anti-microbial films being oriented generally parallel to the incoming radiation. This orientation substantially reduced the level of activation of the anti-microbial coatings, such that no increase in the zone of inhibition was observed relative to controls which had not been irradiated.

Example 16

This example is included to illustrate activation of the anti-microbial coatings in accordance with the present invention with gamma radiation using a dielectric material adjacent to the material during irradiation.

A number of 2.5 cm x 2.5 cm pieces of high density polyethylene mesh (such as used in burn wound dressings) were sputter coated with silver metal under the same conditions as set forth in Example 15 with the exception that the power was 0.1 kW. The coated mesh was then irradiated (perpendicular orientation) as set forth in Example 15 at 4 megarads. The biological activity was then tested, as set out in Example 15. Control mesh samples (silver coated, no irradiation) gave a 10mm ZOI(corrected), while the irradiated samples gave a 14 mm ZOI(corrected).

Further samples of the coated mesh were irradiated while sandwiched between two 2.5 cm x 2.5 cm silicon wafers having a 1000 Å thermally grown oxide layer, as supplied by the Alberta Microelectronics Centre, Edmonton, Alberta. This mesh sample was tested for biological activity and was found to produce a 26 mm ZOI(corrected). Without being bound by the same, it is believed that the silicon wafers provide a source of electrons which are forward scattered to the anti-microbial coatings, further enhancing the anti-microbial effect.

Bulk silver sheet metal was tested to determine whether it could be activated to produce an anti-microbial effect by gamma irradiation. The bulk silver sheet metal samples were annealed at 140°C for 90 minutes in air and then irradiated with a 4 megarad dose. The samples were tested for biological activity, but no ZOI was produced. This result appears to indicate that bulk silver, in its normal ordered crystalline state, has too few atomic defects to be activated in accordance with the process of the present invention.

Example 17

This example is included to illustrate that anti-microbial coatings containing atomic disorder at a level that is insufficient to produce an anti-microbial effect can be further activated by gamma irradiation, in accordance with the present invention.

Silver films were sputtered onto silicon wafers, as set forth in Example 15, except that the gas pressure was reduced from 5.3 Pa (40 mTorr) to 0.7 Pa (5 mTorr), resulting in less atomic disorder in the coatings. The silver films were then irradiated with a 4 Mrad dose of gamma radiation, as in Example 15. The irradiated and control films (not irradiated) were tested for biological activity. The control films produced only 1 mm ZOI(corrected), while the irradiated coatings produced 10 mm ZOI(corrected). This result demonstrates that anti-microbial materials prepared under conditions such that they contain atomic disorder at a level insufficient to produce an anti-microbial effect can be activated so as to be anti-microbial by irradiating with a source of gamma radiation.

Example 18

This example is included to demonstrate the generation of silver complex ions which are distinct from the $Ag^+$ ion and which are highly efficacious in generating an anti-microbial effect. The example provides comparative diffusion and zone of inhibition (ZOI) data for various silver solutions.

Solutions were prepared to generate 10,000 ppm Ag as $AgNO_3$, $Ag(NH_3)_2^+$, $Ag(CN)_2^-$, $Ag(S_2O_3)_2^{3-}$ and Ag(protein).

The silver solutions were prepared as follows:

1) $Ag(S_2O_3)^{3-}$ - 2.66 g of AgCl were dissolved in 150 ml of deionized water. 17.22 g of $Na_2(S_2O_3)$ were added and the volume was brought up to 200 ml with deionized water.

2) $Ag(CN)_2^-$ - Equal volumes of 12.5 g/L AgCN and 50g/L KCN were mixed.

3) Ag(protein) - Two silver protein samples were tested. Silver protein powder (0.5 g of Sigma S-6767, lot # 121H3437, 20% Ag) were added to 10 ml of deionized water. Silver protein powder (1.25 g of Sigma S-9017, lot # 33H3456, 8% Ag) were added to 10 ml of deionized water.

4) $Ag(NH_3)_2^+$ -- Silver nitrate was added to ammonium hydroxide to form a black precipitate. To this solution was added dropwise additional ammonium hydroxide until the precipitate redissolved, leaving the complex silver ion $Ag(NH_3)_2^+$ in solution.

Also prepared were control solutions containing the same concentrations of nitrate, ammonia, cyanide and thiosulphate as was present in the test solutions. The anti-microbial effect of the test solutions was tested by a zone of inhibition test. A sensi disc (cellulose, 6mm diameter) containing 25 microlitres of each of the test solutions was placed in the middle of a MHA (Difco media) plate. The silver complexes or ions in the sensi disc were allowed to diffuse for 4 hours on the MHA plate stored in a 37°C incubator. After 4 hours, the semi disc was removed from the plate and analyzed for silver content using neutron activation analysis (NAA, University of Alberta Slowpoke Reactor Facility). A further set of plates were used to measure zones of inhibition against *S. aureus* for each of the silver complexes or ions in the semi discs. Samples of the agar were taken from the plates from two locations - the edge of the zone of inhibition and underneath the discs. The agar samples were analyzed for silver content by NAA. The control solutions were tested for anti-microbial effect and were found to cause no zone of inhibition. The results are set forth in Table 10.

Table 10

| And-Microbial Effect of Ag$^+$ Ion Compared to Silver Complex Ions | | | | |
|---|---|---|---|---|
| Test Solution | ZOI (mm) | Silver Content (ppm) | | Edge of ZOI |
| | | In Disc | Under Disc | |
| Ag(NO)$_3$ | 6 | 9000 | 100 | 1.8 |
| Ag(NH$_3$)$_2$$^+$ | 18 | 7300 | 221 | 1.7 |
| Ag(CN)$_2$$^-$ | 70 | 1400 | 420 | 4.3 |
| Ag(S$_2$O$_3$)$_2$$^{3-}$ | 36 | * | * | * |
| Ag(protein) | 6 | * | * | * |

\* Not measured

The above results indicate that silver salts or compounds known to dissociate to produce the Ag$^+$ ion (ex. silver nitrate and silver proteins) have a limited anti-microbial effect (6mm ZOI). The anti-microbial effect is greater for silver compositions which release silver complex ions other than Ag$^+$ (ex. Ag(NH$_3$)$_2$$^+$, Ag(CN)$_2$$^-$ and Ag(S$_2$O$_3$)$_2$$^{3-}$). It is also apparent that the silver complex ions are able to diffuse further in the agar medium than the Ag$^+$ ion, thereby achieving an anti-microbial effect further from the silver source.

Without being bound by the same, it is believed that the Ag$^+$ ion is less efficacious in its anti-microbial effect because it readily precipitates in the agar medium with chloride ions known to be present The silver complex ions on the other hand demonstrate a higher level of anti-microbial effect and more rapid diffusion. The silver complex ions are also believed not to precipitate with chloride ions to such an extent, making them more suitable for use in industrial systems or with medical devices and the like which come into contact with fluids containing chloride ions.

Example 19

This example provides comparative diffusion data and zone of inhibition data for several silver anti-microbial coatings.

Three silver films were sputtered under the conditions set forth in Table 11.

TABLE 11

| Sputtering Conditions | Film 1 | Film 2 | Film 3 |
|---|---|---|---|
| Target (20.3 cm dia) | 99.99% Ag | 99.99% Ag | 99.99% Ag |
| Working Gas | 99/1 wt% Ar/O$_2$ | 99/1 wt% Ar/O$_2$ | 99/1 wt% |
| Working Gas Pressure | 0.7 Pa | 5.3 Pa | 5.3 Pa |
| Total Mass Flow | 700 sccm | 700 sccm | 700 sccm |
| Power | 0.5 kW | 0.5 kW | 0.05 kW |
| Substrate Temperature | 21°C | 21°C | 21°C |
| Anode/Cathode Distance | 100 mm | 100 mm | 100 mm |

The coatings were tested for anti-microbial activity by a ZOI test, as set forth in previous examples. Silver content was measured by NAA after 4 hours diffusion in the agar medium, as set forth in Example 18. The comparative results are set out in Table 12.

Table 12

| Anti-Microbial Effect of Silver Coatings | | | | |
|---|---|---|---|---|
| Test Film | Ag Species | CZOI (mm) | Silver Content (ppm) | |
| | | | Under Film | Edge of ZOI |
| Film 1 | $Ag^+$ | 2 | 35 | 0.8 |
| Film 2 | $AgX^1$ | 12 | 8.5 | 0.7 |
| Film 3 | $Ag^+ + AgX^1$ | 12 | 654 | 0.4 |

[1] AgX is a silver complex ion or ion pair.

For Film 1, which releases predominantly $Ag^+$ ions, a small ZOI is produced, with the silver being precipitated as AgCl below the film. For Film 2, a much larger ZOI (6X) is produced with ¼ the amount of silver being precipitated under the wafer. This suggests that a silver complex ion different than $Ag^+$ is formed which diffuses more readily. It is believed that the diffusion is accelerated as a result of the nature of the complex silver species. Film 3 releases much more silver than Films 1 or 2, but the bulk of the silver is in the form of $Ag^+$ which precipitates as AgCl under the film. However, the size of the ZOI indicates that, in addition to $Ag^+$, a complex silver ion with much greater mobility than $Ag^+$ is generated. It is believed that one or more of the negative silver hydroxyl ions $Ag(OH)_2^-$, $Ag_2(OH)_3^-$, or $Ag_3(OH)_4^-$ are generated. In that chloride is in the agar medium, negative silver hydroxyl-chloro complexes may form.

Example 20

This example is included to demonstrate the preparation of complex ions of silver cyanide, and the anti-microbial effect of such ions.

A silver cyanide bath typically used in electroplating was tested for anti-microbial effect using 25 microlitres of bath on a sensi disc in a standard ZOI test. The silver cyanide bath contained 37 g/L silver cyanide, 45 g/L potassium cyanide and 30 g/L potassium carbonate. The resulting ZOI covered the entire plate, indicating a corrected ZOI greater than 94 mm. The maximum amount of silver that was available in the AgCN bath was 30,000 ppm. From previous work it is known that this concentration as $AgNO_3$ would not yield a ZOI greater than 6 mm. The effect of the cyanide ion alone was determined by placing 25 microlitres of 45 g/L KCN on a sensi disc and repeating the ZOI test. A corrected ZOI of 12.5 mm was produced. A solution of AgCN in distilled water (37 g/L) was similarly tested for a ZOI. A corrected ZOI of 14 mm was observed.

The molar ratio of silver ion to cyanide ion in the bath was 0.37:1. This favours the formation of a negative silver cyanide complex $Ag(CN)_2^-$ or AgCN(aq) as an ion pair. The above results demonstrate that these complex silver ions have anti-microbial efficacy and increased mobility within an agar medium.

Thin strips of filter paper were treated with 50 microlitres of either a silver nitrate solution (10,000 ppm Ag) or a potassium cyanide solution (6,400 ppm $CN^-$). The strips were subjected to a standard ZOI test on the MHA plate. Silver nitrate control strips gave a corrected ZOI of 8 mm, while the KCN control strips gave no ZOI. When one of each of the silver nitrate and potassium cyanide strips were placed on the MHA plate at right angles to each other, the corrected ZOI was 30 mm from the silver nitrate strip and 22 mm from the potassium cyanide strip.

This result demonstrates that a complex silver ion resulting from the combination of silver nitrate and potassium cyanide in the media has greater anti-microbial efficacy than either solution alone.

Example 21

This example is included to demonstrate the anti-microbial efficacy of a complex silver ion of silver chloride.

Silver chloride was pressed into a 0.2 g pellet at 413,550 kPa (60,000 psi) and tested using a standard ZOI test on MHA plates. An 8 mm zone resulted. A mixture of 0.15g AgCl and 0.05 g NaCl was pressed into a pellet at 60,000 psi and similarly tested. A 24 mm zone was observed.

The increased concentration of the available chloride ion favours the formation of the complex silver ion $AgCl_2^-$, which is demonstrated above to have improved anti-microbial efficacy over AgCl.

A silver nitrate solution (10,000 ppm Ag) was tested with sensi discs (25 microlitres) in a ZOI test. A 6 mm zone was observed. The same concentration of $AgNO_3$ was tested on an agar plate which had been supplemented with 5% NaCl. A 20 mm zone was observed, indicating improved anti-microbial efficacy. A control plate of agar supplemented with 5% NaCl did not inhibit bacterial growth (*S. aureus*).

It is believed that the higher concentrations of the chloride ion favoured the formation of the complex silver ion $Ag(Cl)_2^-$. This species shows three times the anti-microbial efficacy of $Ag^+$ from silver nitrate.

Example 22- Animal Testing - Irritation

A primary skin irritation study was performed on New Zealand White (NZW) rabbits using gauze coated with an anti-microbial metal of this invention. The coating was deposited on a USP type VII gauze using the process conditions of example 7 where the working gas was 99/1 wt% $Ar/O_2$.

The coated gauze was placed on abraded and unabraded skin on the side of a New Zealand White rabbit. At 24 h the gauze was removed and the site was graded for erythema and edema at 1, 24 and 48 hours after removal.

All animals survived to the end of the study. No erythema, edema or infection was observed on any animal. It was concluded that the gauze did not produce local irritation when placed on the skin of male or female NZW rabbits.

Example 23 - Animal Testing - Sensitivity

The sensitivity of Hartley Guinea Pigs to USP type VII gauze coated with an anti-microbial metal coating of the present invention was investigated. The gauze was coated as per Example 7 using 99/1 wt% $Ar/O_2$. The spilt adjuvant technique was used since the test material was not injectable and the application of dry ice to the induction area most closely simulates the clinical situation.

There was no evidence that the coated gauze induced erythema or edema and no infection was observed in any of the animals. All animals survived the study.

Application of the coated gauze to the skin of male Hartley Guinea Pigs did not result in local sensitivity when tested by the split adjuvant technique.

Example 24

This example is included to demonstrate that silver powder/NaCl mixtures produce an anti-microbial effect from complex silver ions believed to be $AgCl_2^-$.

Pellets of silver powder (1 micron) and NaCl (25%) were pressed at the conditions set out below. The anti-microbial effect was measured by a zone of inhibition test with the pellets. A comparative control of pressed silver powder was also tested for a zone of inhibition. The results are shown in Table 13:

Table 13

| Anti-Microbial Effect of Silver Powder/NaCl | | |
|---|---|---|
| Pellet | Compression kg (lb.) | ZOI |
| Ag + 25% NaCl | 454 (1000) | 26 mm |
| Ag + 25% NaCl | 1361 (3000) | 20 mm |
| Ag + 25% NaCl | 2268 (5000) | 19 mm |
| Ag powder | 454 (1000) | <1 mm |

Example 25

This example illustrates the structural and chemical characteristics of sputter deposited silver films that exhibit good anti-microbial activity (corrected zone of inhibition, CZOI) using the zone of inhibition test as set forth in previous examples. The films were produced by sputtering of a solid 20.3 cm dia planar silver magnetron target onto silicon wafer substrates (100 mm from the target) under the conditions summarized in Table 14. The total mass gas flow was 700 sccm. The ratio of substrate temperature to melting point of silver (1234K), $T/T_m$, was less than 0.3, the thickness of the film was nominally 3000Å and the angle of incidence in each case was 90° (normal incidence). The characteristics of as deposited silver as well as those that were subsequently annealed (in air at 140°C for 90 minutes) are described in this example. The films were characterized in terms of structural (grain size, type of defects, recrystallization) and chemical properties (dopant concentration (wherein dopant refers to atomic %O or oxide content), and electrochemical rest potential). The results are summarized in Tables 15 and 16.

The dopant concentration in the film was measured using x-ray photoelectron spectroscopy (XPS) and secondary ion mass spectrometry (SIMS). In the XPS technique a monochromatized Al Kα x-ray beam was used as the incident beam. A 4kV Ar ion beam was rastered over a 2 mm x 2 mm area in order to remove surface contaminants and expose a fresh surface for XPS analysis. A positive cesium ion beam at 12.5 kV was employed for the SIMS analysis. The dopant concentration computed from XPS and SIMS data is summarized in Tables 15 and 16 for both as deposited and annealed films. It can be seen that one preferred characteristic of biologically active silver films in accordance with the invention is the presence of a dopant. The XPS and SIMS data further showed that the dopant, which in the present case was oxygen or both silver oxide and oxygen, was not chemically bound to the silver atoms in the bulk film. Moreover, the dopant as oxygen was incorporated in such amounts as to exceed the room temperature solid solubility in silver.

The grain size of as deposited and annealed films was measured from images taken with a transmission electron microscope (TEM). These data, reported in Tables 10 and 11, demonstrate that anti-microbial active silver films of this invention have an average grain size smaller than 200 nm. Active films, as deposited, had an average grain size less than about 140 nm. The most active films, as deposited, had an average grain size less than 90 nm. In addition, high resolution transmission electron microscopy showed that the onset of recrystallization (Trec) commenced at about 90°C. Grain growth of these fine grained, biologically active films, occurred at temperatures well below 0.33 $T_m$, where $T_m$ is the melting point of silver in degrees K, in particular below 140°C. In general, recrystallization diminished anti-microbial activity. However, coatings with higher levels of silver oxide (coatings 3 and 6) retained anti-microbial activity after annealing. It is believed that the oxide pins sufficient atomic defects so as to retain anti-microbial activity after annealing.

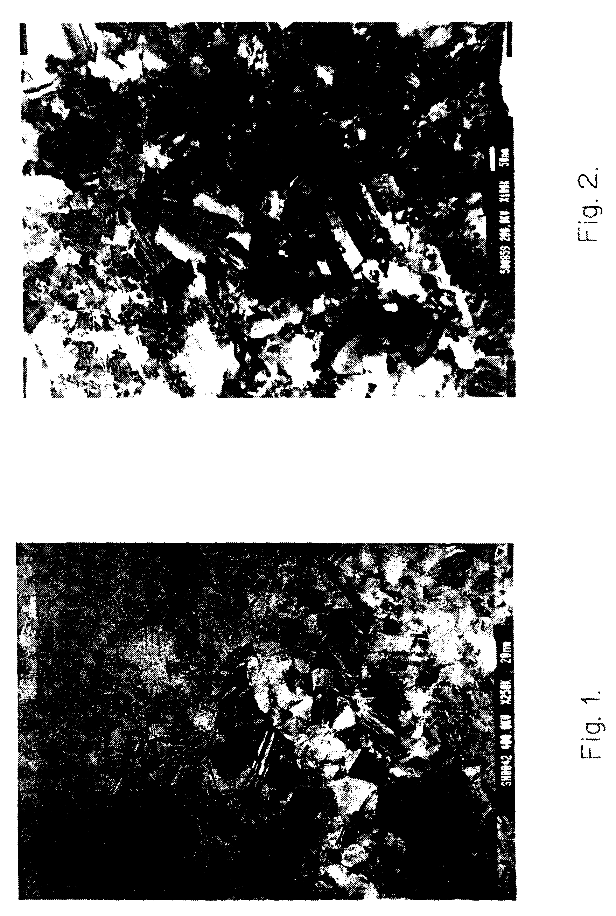

The TEM analysis further indicated that biologically active silver films contained a number of growth twins. Upon annealing in air at 140°C for 90 minutes these growth twins disappeared and annealing twins appeared. These latter twins were, however, the result of recovery, recrystallization and grain growth which transformed the silver film into a lower energy state. Evidently, these deposited silver films, along with the associated growth twins that underwent such grain growth, were in a higher energy state. Thus, the presence of these aforementioned defects in the as deposited films is a distinguishing characteristic of anti-microbial coatings in accordance with this invention. Figures 1 and 2 are TEM micrographs showing the grain sizes and twins observed in as deposited and annealed silver films respectively.

The rest potential of the silver films was measured in one molar (1M) potassium hydroxide (KOH) solution using a saturated calomel electrode (SCE) as the reference electrode. Tables 15 and 16 show that the silver films exhibited anti-microbial behaviour only when the rest potential was positive. No biological activity was observed when the rest potential was negative.

Table 14

| Growth Conditions for Sputter Deposited Silver Anti-microbial Coatings | | | |
|---|---|---|---|
| ID Number | GROWTH CONDITIONS | | |
| | Gas Composition | Pressure Pa (mTorr) | Power(kW) |
| 1 | 99% Ar, 1% O | 1.3 (10) | 0.10 |
| 2 | 99% Ar, 1% O | 1.3 (10) | 0.50 |
| 3 | 99% Ar, 1% O | 5.3 (40) | 0.05 |
| 4 | 99% Ar, 1% O | 5.3 (40) | 0.10 |
| 5 | 99% Ar, 1% O | 5.3 (40) | 0.50 |
| 6 | 80% Ar, 20% O | 5.3 (40) | 0.10 |

Table 15

| Structural Characteristics of Sputter Deposited Silver Anti-microbial Coatings | | | | | |
|---|---|---|---|---|---|
| Growth Condition ID Number | As Deposited | | | | |
| | Grain Size (nm) | Depot Concentration Atomic %O | Rest Potential mV (vs SCE)[1] | Defects | CZOI (mm) |
| 1 | 37 | 5.5 | +125 | Growth twins | 9 |
| 2 | 148 | 0 | -342 | - | 2 |
| 3 | 21 | 20.0* | +150 | Growth twins | 10 |
| 4 | 19 | 8.0 | +135 | Growth twins | 7 |
| 5 | 41 | 3.4 | +131 | Growth twins | 9 |
| 6 | 22 | 58.0* | +146 | - | 8 |
| Bulk Silver | >200 | 0 | -170 | - | <1 |

\* as $Ag_2O$
[1] These values are subject to variability of $\pm$ 20 mV
- not measured

Table 16

| Structural Characteristics of Annealed Silver Anti-microbial Coatings | | | | | |
|---|---|---|---|---|---|
| Growth Condition ID Number | Annealed at 140°C, 90 Minutes | | | | |
| | Grain Size (nm) | Dopant Concentration atomic %O | Rest Potential mV (vs SCE)[1] | Defects | CZOI (mm) |
| 1 | 91 | - | -6 | Annealing twins | 1 |
| 2 | 135 | 0 | -224 | Annealing twins | 0 |
| 3 | 130 | 16.0* | +121 | Annealing twins | 10 |
| 4 | 73 | 0.8 | +33 | Annealing twins | 8 |
| 5 | 132 | 0.7 | -29 | Annealing twins | 0 |
| 6 | - | 31.0* | +127 | - | 8 |
| Bulk Silver | >200 | 0 | -170 | - | <1 |

\* as $Ag_2O$
[1] These values are subject to variability of $\pm$20 mV
- not measured

All publications mentioned in this specification are indicative of the level of skill of those skilled in the art to which this invention pertains.

The terms and expressions in this specification are used as terms of description and not of limitation. There is no intention, in using such terms and expressions, of excluding equivalents of the features illustrated and described, it being recognized that the scope of the invention is defined and limited only by the claims which follow.

**Claims**

1. A method of forming an anti-microbial material containing one or more anti-microbial metals, said method comprising:

creating atomic disorder in a crystalline material containing one or more anti-microbial metals under conditions which limit diffusion for retaining atomic disorder therein to provide sustained release of atoms, ions, molecules or clusters of at least one of the metals into an alcohol or water based electrolyte at an enhanced rate relative to the material in its normal ordered crystalline state; and
irradiating the material with a low linear energy transfer form of radiation to release at least one anti-microbial metal at a concentration sufficient to provide a localized anti-microbial effect.

2. The method as set forth in claim 1, wherein the anti-microbial metal is selected from the group consisting of Ag, Au, Pt, Pd, Ir, Sn, Cu, Sb, Bi. Zn, alloys thereof and compounds thereof.

3. The method as set forth in claim 2, wherein the material is a powder or foil of one or more of the anti-microbial metals, and wherein the atomic disorder is formed by cold working of the powder or foil.

4. The method as set forth in claim 3, wherein the material is a nanocrystalline powder.

5. The method as set forth in claim 2, wherein the material is formed as a coating on a substrate by vapour deposition under conditions which limit diffusion during deposition and which limit annealing or recrystallization following deposition.

6. The method as set forth in claim 5, wherein the material is formed by vacuum evaporation, sputtering, magnetron sputtering or ion plating.

7. The method as set forth in claim 6, wherein the anti-microbial material is a composite coating formed by co-, sequentially or reactively depositing an anti-microbial metal in a matrix with atoms or molecules of a different material to create atomic disorder in the matrix, said different material being deposited as one or more members selected from the group consisting of oxygen, nitrogen, hydrogen, boron, sulphur or halogen absorbed or trapped in the matrix from the atmosphere of the vapour deposition; an oxide, nitride, carbide, boride, halide, sulphide or hydride of an anti-microbial metal; and an oxide, nitride, carbide, boride, halide, sulphide or hydride of an inert biocompatible metal selected from the group consisting of Ta. Ti, Nb, V. Hf, Zn, Mo, Si, and Al.

8. The method as set forth in claim 7, wherein the anti-microbial metal is silver and said different material is one or both of silver oxide and atoms or molecules containing oxygen trapped or absorbed in the matrix from the atmosphere of the vapour deposition.

9. The method as set forth in claim 5, wherein the coating is formed by magnetron sputtering at conditions such that the ratio of the temperature of the surface being coated to the melting point of the anti-microbial material being deposited, in degrees K, is less than about 0.5, and the working gas pressure is greater than about 1.3 Pa (10mT).

10. The method as set forth in claim 7, wherein the coating is formed by magnetron sputtering at conditions such that the ratio of the temperature of the surface being coated to the melting point of the anti-microbial material being deposited, in degrees K, is less than about 03, and the working gas pressure is greater than about 1.3 Pa (10mT).

11. The method as set forth in claim 8, wherein the coating is formed by magnetron sputtering at conditions such that the ratio of the temperature of the surface being coated to the melting point of the anti-microbial material being deposited, in degrees K, is less than about 0.5, and the working gas pressure is greater than about 1.3 Pa (10mT).

12. The method as set forth in claim 1, 3 or 6, wherein the form of radiation is selected from gamma, beta and x-rays.

13. The method as set forth in claim 1, 3 or 6, wherein the source of radiation is gamma radiation, used at a dose of greater than about 1 Mrad.

14. The method as set forth in claim 1, 3 or 6, wherein the anti-microbial material being irradiated is oriented substantially perpendicular to the incoming radiation.

15. The method as set forth in claim 1, 3 or 6, wherein the material is placed adjacent to a dielectric material during irradiation.

**16.** The method as set forth in claim 1, 3 or 6, wherein the material is sandwiched between silicon oxide surfaces during irradiation.

**17.** A fine grain anti-microbial material, comprising:

one or more anti-microbial metals, or alloys or compounds thereof in the form of a fine grain crystalline powder, having a grain size less than 200 nm, characterized by sufficient atomic disorder such that the material, in contact with an alcohol or a water based electrolyte, provides a sustained release of atoms, ions, molecules or clusters containing at least one metal at a concentration sufficient to provide a localized anti-microbial effect, wherein the anti-microbial metal is formed in a matrix with atoms or molecules of a different material, the different material being selected from inert biocompatible metals, oxygen, nitrogen, hydrogen, boron, sulphur, halogen, and oxides, nitrides, carbides, borides, sulphides and halides of either or both of an anti-microbial metal or an inert biocompatible metal.

**18.** The anti-microbial material as set forth in claim 17, wherein the anti-microbial metal is selected from the group consisting of Ag, Au, Pt, Pd, Ir, Sn, Cu. Sb, Bi, and Zn or an alloy or compound thereof, and wherein the inert biocompatible metal is selected from the group consisting of Ta, Ti, Nb, B, Hf, Zn, Mo, Si, and Al.

**19.** The anti-microbial material as set forth in claim 18, wherein the anti-microbial metal is selected from Ag, Au or Pd, and wherein the inert biocompatible metal is selected from Ta, Ti or Nb.

**20.** The anti-microbial material as set forth in claim 17, comprising substantially pure silver metal, silver oxide and trapped or absorbed atoms of oxygen.

**21.** The anti-microbial material as set forth in claim 17, 18, 19, or 20 in the form of a nanocrystalline powder having a grain size less than about 20 nm, or in the form of a fine grain powder having a grain size less than about 140 nm.

**22.** The anti-microbial material as set forth in claim 17 wherein the anti-microbial metal is silver, or an alloy or compound thereof, in a crystalline form, and wherein the material is characterized as having a positive rest potential, when measured against a saturated calomel reference electrode, in 1M potassium hydroxide and having a ratio of its temperature of recrystallization to its melting temperature, in degrees K, (Trec/Tm), loss than 0.33, and which, in contact with an alcohol or a water based electrolyte, releases atoms, ions, molecules or clusters containing silver or a sustained basis at a concentration sufficient to provide a localized anti-microbial effect.

**23.** The material as set forth in claim 22, wherein the material is further characterized in that the ratio of its temperature of recrystallization to its melting temperature, in degrees K, ($T_{rec}/T_m$), is less than about 0.3

**24.** The material as set forth in claim 22, wherein the material is further characterized in that it has a temperature of recrystallization less than about 140°C.

**25.** The material as set forth in claim 24, wherein the material is further characterized in that it has a grain size less than about 200nm, or wherein the material is further characterized in that it has a grain size less than about 140 mm e.g. less than 90 nm.

**26.** The material as set forth in claim 25, in the form of a nanocrystalline powder.

**27.** The material as set forth in claim 26, in the form (a) substantially pure silver metal, or (b) a mixture of substantially pure silver metal and silver oxide, or (c) substantially pure silver metal and absorbed, trapped, or reacted atoms or molecules of oxygen, and optionally also including silver oxide.

**28.** A method of producing a fine grain anti-microbial material, characterized by:

depositing one or more anti-microbial metals in a matrix with atoms or molecules of a different material, in a powder form, by vapour deposition onto a cooled substrate, to provide a crystalline material having atomic disorder such that the powder, in contact with an alcohol or a water based electrolyte, provides a sustained release of ions, atoms, molecules or clusters of at least one of the anti-microbial metals into the alcohol or water based electrolyte at a concentration sufficient to provide a localized anti-microbial effect, wherein the different material is selected from the group consisting of inert biocompatible metals, oxygen, nitrogen, hydrogen, boron, sulphur, halogens, and oxides, nitrides, carbides, borides, sulphides and halides of either or both of an anti-microbial metal or an inert biocompatible metal.

29. The method as set forth in claim 28, wherein the anti-microbial metal is selected from the group consisting of Ag, Au, Pr, Pd, Ir, Sn, Cu, Sb, Bi and Zn or alloys or compounds of one or more of these metals, and wherein the inert biocompatible metal is selected from the group consisting of Ta, Ti, Nb, B, Hf, Zn, Mo, Si and Al or alloys or compounds of one or more of these metals.

30. The method as set forth in claim 29, wherein the anti-microbial metal is selected from Ag, Au, and Pd, and wherein the inert biocompatible metal is selected from Ta, Ti, and Nb.

31. The method as set forth in claim 30, wherein oxygen is included in the working gas atmosphere during vapour deposition such that atoms or molecules of oxygen are trapped or absorbed in the matrix.

32. The method as set forth in claim 31, wherein the anti-microbial metal which is deposited is substantially pure silver metal or silver oxide and wherein oxygen may be included in the working gas atmosphere such that the deposited material includes substantially pure silver metal, and one or both of silver oxide and atoms or molecules of trapped or absorbed oxygen.

33. The method as set forth in claim 30, 31, or 32, wherein the material is deposited as a fine grain powder having a grain size less than about 200 nm, preferably less than 140 mm eg. a nanocrystalline powder having a grain size less than about 20 nm.

**Patentansprüche**

1. Verfahren zur Bildung eines antimikrobiellen Materials, enthaltend ein oder mehrere antimikrobielle Metalle, wobei das Verfahren folgendes umfaßt:

   Erzeugen von atomarer Unordnung in einem kristallinen Material, enthaltend ein oder mehrere antimikrobielle Metalle, unter Bedingungen, welche die Diffusion limitieren zur Beibehaltung der atomaren Unordnung darin, um eine verzögerte Freisetzung von Atomen, Ionen, Molekülen oder Clustern von mindestens einem der Metalle in einen Elektrolyten auf Alkohol- oder Wasserbasis bei einer gesteigerten Rate im Verhältnis zu dem Material in seinem normalen geordneten kristallinen Zustand vorzusehen; und

   Bestrahlen des Materials mit einer niedrigen linearen Energietransfer-Form von Strahlung, um mindestens ein antimikrobielles Metall bei einer ausreichenden Konzentration freizugeben, um einen lokalisierten antimikrobiellen Effekt bereitzustellen.

2. Verfahren, wie dargelegt in Anspruch 1, worin das antimikrobielle Metall gewählt wird aus der Gruppe, bestehend aus Ag, Au, Pt, Pd, Ir, Sn, Cu, Sb, Bi, Zn, Legierungen davon und Verbindungen davon.

3. Verfahren, wie dargelegt in Anspruch 2, worin das Material ein Pulver oder eine Folie aus einem oder mehreren der antimikrobiellen Metalle ist, und wobei die atomare Unordnung durch Kaltbearbeiten des Pulvers oder der Folie gebildet wird.

4. Verfahren, wie dargelegt in Anspruch 3, worin das Material ein nanokristallines Pulver ist.

5. Verfahren, wie dargelegt in Anspruch 2, worin das Material als Überzug auf einem Substrat durch Dampfabscheidung unter Bedingungen gebildet wird, welche die Diffusion während der Abscheidung limitieren und welche das Tempern oder die Umkristallisation im Anschluß an die Abscheidung limitieren.

6. Verfahren, wie dargelegt in Anspruch 6, worin das Material gebildet wird durch Vakuumaufdampfen, Sputtern, Magnetron-Sputtern oder Ionenplattierung.

7. Verfahren, wie dargelegt in Anspruch 5, worin das antimikrobielle Material ein Verbundüberzug ist, gebildet durch Co-, sequenzielles oder reaktives Abscheiden eines antimikrobiellen Metalls in einer Matrix mit Atomen oder Molekülen eines unterschiedlichen Materials, um atomare Unordnung in der Matrix zu erzeugen, wobei das unterschiedliche Material als ein oder mehrere Vertreter abgeschieden wird, gewählt aus der Gruppe, bestehend aus Sauerstoff, Stickstoff, Wasserstoff, Bor, Schwefel oder Halogen, absorbiert oder eingefangen in der Matrix aus der Atmosphäre der Dampfabscheidung; eines Oxids, Nitrids, Carbids, Borids, Halogenids, Sulfids oder Hydrids eines antimikrobiellen Metalls; und eines Oxids, Nitrids, Carbids, Borids, Halogenids, Sulfids oder Hydrids eines inerten biokompatiblen Metalls, gewählt aus der Gruppe, bestehend aus Ta, Ti, Nb, V, Hf, Zn, Mo, Si und Al.

8. Verfahren, wie dargelegt in Anspruch 7, worin das antimikrobielle Metall Silber ist und das unterschiedliche Material eines oder beides aus Silberoxid und Atomen oder Molekülen ist, welche Sauerstoff aus der Atmosphäre der Dampfabscheidung eingefangen oder absorbiert in der Matrix enthalten.

9. Verfahren, wie dargelegt in Anspruch 5, wobei der Überzug durch Magnetron-Sputtern bei solchen Bedingungen gebildet wird, daß das Verhältnis der Temperatur der Oberfläche, welche beschichtet wird, zu dem Schmelzpunkt des antimikrobiellen Materials, das abgeschieden wird, in Grad Kelvin, geringer ist als etwa 0,5, und der Arbeits-Gasdruck größer ist als etwa 1,3 Pa (10 mT).

10. Verfahren, wie dargelegt in Anspruch 7, wobei der Überzug durch Magnetron-Sputtern bei solchen Bedingungen gebildet wird, daß das Verhältnis der Temperatur der Oberfläche, welche beschichtet wird, zu dem Schmelzpunkt des antimikrobiellen Materials, das abgeschieden wird, in Grad Kelvin, geringer ist als etwa 0,5, und der Arbeits-Gasdruck größer ist als etwa 1,3 Pa (10 mT).

11. Verfahren, wie dargelegt in Anspruch 8, wobei der Überzug durch Magnetron-Sputtern bei solchen Bedingungen gebildet wird, daß das Verhältnis der Temperatur der Oberfläche, welche beschichtet wird, zu dem Schmelzpunkt des antimikrobiellen Materials, das abgeschieden wird, in Grad Kelvin, geringer ist als etwa 0,5, und der Arbeits-Gasdruck größer ist als etwa 1,3 Pa (10 mT).

12. Verfahren, wie dargelegt in Anspruch 1, 3 oder 6, wobei die Form der Strahlung unter Gamma-, Beta-, und Röntgenstrahlen gewählt wird.

13. Verfahren, wie dargelegt in Anspruch 1, 3 oder 6, wobei die Strahlungsquelle Gammastrahlung ist, angewandt bei einer Dosis von mehr als etwa 1 MRad.

14. Verfahren, wie dargelegt in Anspruch 1, 3 oder 6, wobei das bestrahlte antimikrobielle Material im wesentlichen senkrecht zur einfallenden Strahlung orientiert ist.

15. Verfahren, wie dargelegt in Anspruch 1, 3 oder 6, wobei das Material während der Bestrahlung angrenzend an ein dielektrisches Material plaziert ist.

16. Verfahren, wie dargelegt in Anspruch 1, 3 oder 6, wobei das Material während der Bestrahlung zwischen Siliciumoxidoberflächen sandwichartig eingefaßt ist.

17. Feinkörniges antimikrobielles Material, umfassend: ein oder mehrere antimikrobielle Metall(e) oder Legierungen oder Verbindungen davon in der Form eines feinkörnigen kristallinen Pulvers mit einer Korngröße von weniger als 200 nm, gekennzeichnet durch ausreichende atomare Unordnung, so daß das Material, im Kontakt mit einem Elektrolyten auf Alkohol- oder Wasserbasis, eine verzögerte Freisetzung von Atomen, Ionen, Molekülen oder Clustern, enthaltend mindestens ein Metall bei einer ausreichenden Konzentration, um einen lokalisierten antimikrobiellen Effekt vorzusehen, bereitstellt, wobei das antimikrobielle Metall in einer Matrix mit Atomen oder Molekülen eines unterschiedlichen Materials geformt ist, wobei das unterschiedliche Material gewählt ist aus inerten biokompatiblen Metallen, Sauerstoff, Stickstoff, Wasserstoff, Bor, Schwefel, Halogen, und Oxiden, Nitriden, Carbiden, Boriden, Sulfiden und Halogeniden von einem oder beiden eines antimikrobiellen Metalls oder eines inerten biokompatiblen Metalls.

18. Antimikrobielles Material, wie dargelegt in Anspruch 17, wobei das antimikrobielle Metall gewählt ist aus der Gruppe, bestehend aus Ag, Au, Pt, Pd, Ir, Sn, Cu, Sb, Bi und Zn oder einer Legierung oder Verbindung davon, und wobei das inerte biokompatible Metall gewählt ist aus der Gruppe, bestehend aus Ta, Ti, Nb, B, Hf, Zn, Mo, Si und Al.

19. Antimikrobielles Material, wie dargelegt in Anspruch 18, wobei das antimikrobielle Metall gewählt ist aus Ag, Au oder Pd, und wobei das inerte biokompatible Metall gewählt ist aus Ta, Ti oder Nb.

20. Antimikrobielles Material, wie dargelegt in Anspruch 17, umfassend im wesentlichen reines Silbermetall, Silberoxid und eingefangene oder absorbierte Sauerstoffatome.

21. Antimikrobielles Material, wie dargelegt in Anspruch 17, 18, 19 oder 20, in der Form eines nanokristallinen Pulvers mit einer Korngröße von weniger als etwa 20 nm oder in der Form eines feinkörnigen Pulvers mit einer Korngröße von weniger als etwa 140 nm.

22. Antimikrobielles Material, wie dargelegt in Anspruch 17, wobei das antimikrobielle Metall Silber oder eine Legierung oder Verbindung davon in einer kristallinen Form ist, und wobei das Material dadurch gekennzeichnet ist, daß es ein positives Ruhepotential aufweist, wenn gegen eine gesättigte Kalomel-Referenzelektrode gemessen wird, in 1 M Kaliumhydroxid, und ein Verhältnis von seiner Umkristallisierungs-Temperatur zu seiner Schmelztemperatur in Grad Kelvin ($T_{rec}/T_m$) von weniger als 0,33 aufweist, und das bei Kontakt mit einem Elektrolyten auf Alkohol- oder Wasserbasis Atome, Ionen, Moleküle oder Cluster, welche Silber enthalten, auf einer verzögerten Basis in einer ausreichenden Konzentration freisetzt, um einen lokalisierten antimikrobiellen Effekt vorzusehen.

23. Material, wie dargelegt in Anspruch 22, wobei das Material ferner dadurch gekennzeichnet ist, daß das Verhältnis von seiner Umkristallisierungs-Temperatur zu seiner Schmelztemperatur in Grad Kelvin ($T_{rec}/T_m$) weniger als etwa 0,3 beträgt.

24. Material, wie dargelegt in Anspruch 22, wobei das Material ferner dadurch gekennzeichnet ist, daß es eine Umkristallisierungs-Temperatur von weniger als etwa 140°C aufweist.

25. Material, wie dargelegt in Anspruch 24, wobei das Material ferner dadurch gekennzeichnet ist, daß es eine Korngröße von weniger als etwa 200 nm aufweist, oder wobei das Material ferner dadurch gekennzeichnet ist, daß es eine Korngröße von weniger als etwa 140 nm, z.B. weniger als 90 nm, aufweist.

26. Material, wie dargelegt in Anspruch 25, in der Form eines nanokristallinen Pulvers.

27. Material, wie dargelegt in Anspruch 26, in der Form (a) von im wesentlichen reinem Silbermetall oder (b) eines Gemischs von im wesentlichen reinem Silbermetall und Silberoxid oder (c) von im wesentlichen reinem Silbermetall und absorbierten, eingefangenen oder reagierten Atomen oder Molekülen von Sauerstoff, und gegebenenfalls auch einschließlich Silberoxid.

28. Verfahren zur Herstellung eines feinkörnigen antimikrobiellen Materials, gekennzeichnet durch:

Abscheiden eines oder mehrerer antimikrobieller Metalle in einer Matrix mit Atomen oder Molekülen eines unterschiedlichen Materials, in einer Pulverform, durch Dampfabscheidung auf ein gekühltes Substrat, um ein kristallines Material mit atomarer Unordnung bereitzustellen, so daß das Pulver in Kontakt mit einem Elektrolyten auf Alkohol- oder Wasserbasis eine verzögerte Freisetzung von Ionen, Atomen, Molekülen oder Clustern von mindestens einem der antimikrobiellen Metalle in den Elektrolyten auf Alkohol- oder Wasserbasis bei einer ausreichenden Konzentration vorsieht, um einen lokalisierten antimikrobiellen Effekt bereitzustellen, wobei das unterschiedliche Material gewählt wird aus der Gruppe, bestehend aus inerten biokompatiblen Metallen, Sauerstoff, Stickstoff, Wasserstoff, Bor, Schwefel, Halogenen und Oxiden, Nitriden, Carbiden, Boriden, Sulfiden und Halogeniden von einem oder beiden eines antimikrobiellen Metalls oder eines inerten biokompatiblen Metalls.

29. Verfahren, wie dargelegt in Anspruch 28, wobei das antimikrobielle Metall gewählt wird aus der Gruppe, bestehend aus Ag, Au, Pt, Pd, Ir, Sn, Cu, Sb, Bi und Zn oder Legierungen oder Verbindungen von einem oder mehreren dieser Metalle, und wobei das inerte biokompatible Metall gewählt wird aus der Gruppe, bestehend aus Ta, Ti, Nb, B, Hf, Zn, Mo, Si und Al oder Legierungen oder Verbindungen von einem oder mehreren dieser Metalle.

30. Verfahren, wie dargelegt in Anspruch 29, wobei das antimikrobielle Metall gewählt wird aus Ag, Au und Pd, und wobei das inerte biokompatible Metall gewählt wird aus Ta, Ti und Nb.

31. Verfahren, wie dargelegt in Anspruch 30, wobei Sauerstoff in der Arbeitsgasatmosphäre während der Dampfabscheidung eingeschlossen ist, so daß Atome oder Moleküle von Sauerstoff in der Matrix eingefangen oder absorbiert werden.

**32.** Verfahren, wie dargelegt in Anspruch 31, wobei das antimikrobielle Metall, das abgeschieden wird, im wesentlichen reines Silbermetall oder Silberoxid ist und wobei Sauerstoff in der Arbeitsgasatmosphäre eingeschlossen sein kann, so daß das abgeschiedene Material im wesentlichen reines Silbermetall und eines oder beides von Silberoxid und Atomen oder Molekülen von eingefangenem oder absorbiertem Sauerstoffeinschließt.

**33.** Verfahren, wie dargelegt in Anspruch 30, 31 oder 32, wobei das Material als ein feinkörniges Pulver mit einer Korngröße von weniger als etwa 200 nm, vorzugsweise weniger als 140 nm, z.B. ein nanokristallines Pulver mit einer Korngröße von weniger als etwa 20 nm, abgeschieden wird.

**Revendications**

**1.** Procédé de formation d'un matériau anti-microbien contenant un ou plusieurs métaux anti-microbiens, ledit procédé comprenant :

la création d'un désordre atomique dans un matériau cristallin contenant un ou plusieurs métaux anti-microbiens dans des conditions qui limitent la diffusion pour y conserver le désordre atomique afin de fournir une libération prolongée d'atomes d'ions de molécules ou d'amas de l'un au moins des métaux dans un alcool ou un électrolyte aqueux à une vitesse supérieure par rapport à ce qu'elle serait s'agissant du matériau dans son état cristallin normalement ordonné ; et
l'irradiation du matériau à l'aide d'une forme de transfert de rayonnement d'énergie linéaire faible pour libérer au moins un métal anti-microbien à une concentration suffisante pour fournir un effet anti-microbien localisé.

**2.** Procédé selon la revendication 1, dans lequel le métal anti-microbien est choisi dans le groupe consistant en Ag, Au, Pt, Pd, Ir, Sn, Cu, Sb, Bi, Zn, leurs alliages et les composés en contenant.

**3.** Procédé selon la revendication 2, dans lequel le matériau est une poudre ou une feuille mince d'un ou plusieurs métaux anti-microbiens et dans lequel le désordre atomique est formé par travail à froid de la poudre ou de la feuille mince.

**4.** Procédé selon la revendication 3, dans lequel le matériau est une poudre nanocristalline.

**5.** Procédé selon la revendication 2, dans lequel le matériau est formé en tant que revêtement sur un substrat par dépôt en phase vapeur dans des conditions qui limitent la diffusion au cours du dépôt et qui limitent la trempe ou la recristallisation à la suite du dépôt.

**6.** Procédé selon la revendication 5, dans lequel le matériau est formé par évaporation sous vide, pulvérisation, pulvérisation par magnétron ou placage ionique.

**7.** Procédé selon la revendication 6, dans lequel le matériau anti-microbien est un revêtement composite formé par co-dépôt, dépôt séquentiel ou dépôt réactif d'un métal anti-microbien dans une matrice avec des atomes ou des molécules d'un matériau différent pour créer un désordre atomique dans la matrice, ledit matériau différent étant déposé sous la forme d'un ou plusieurs éléments choisis dans le groupe consistant en l'oxygène, l'azote, l'hydrogène, le bore, le soufre ou un halogène absorbé ou piégé dans la matrice depuis l'atmosphère du dépôt en phase vapeur ; un oxyde, un nitrure, un carbure, un borure, un halogénure, un sulfure ou un hydrure d'un métal anti-microbien ; et un oxyde, un nitrure, un carbure, un borure, un halogénure, un sulfure ou un hydrure d'un métal inerte biocompatible choisi dans le groupe consistant en Ta, Ti, Nb, V. Hf, Zn, Mo, Si et Al.

**8.** Procédé selon la revendication 7, dans lequel le métal anti-microbien est l'argent et ledit matériau différent est soit l'oxyde d'argent, soit des atomes ou des molécules contenant de l'oxygène, soit les deux, piégés ou absorbés dans la matrice à partir de l'atmosphère du dépôt en phase vapeur.

**9.** Procédé selon la revendication 5, dans lequel le revêtement est formé par pulvérisation par magnétron dans des conditions telles que le rapport entre la température de la surface en cours de revêtement et le point de fusion du matériau anti-microbien en cours de dépôt, exprimé en degrés K, est inférieur à environ 0,5, et la pression du gaz de travail est supérieure à environ 1,3 Pa (10 mT).

**10.** Procédé selon la revendication 7, dans lequel le revêtement est formé par pulvérisation par magnétron dans des conditions telles que le rapport entre la température de la surface en cours de revêtement et le point de fusion du matériau anti-microbien en cours de dépôt, exprimé en degrés K, est inférieur à environ 0,5, et la pression du gaz de travail est supérieure à environ 1,3 Pa (10 mT).

11. Procédé selon la revendication 8, dans lequel le revêtement est formé par pulvérisation par magnétron dans des conditions telles que le rapport entre la température de la surface en cours de revêtement et le point de fusion du matériau anti-microbien en cours de dépôt, exprimé en degrés K, est inférieur à environ 0,5, et la pression du gaz de travail est supérieure à environ 1,3 Pa (10 mT).

12. Procédé selon la revendication 1, 3 ou 6, dans lequel la forme de rayonnement est choisie parmi les rayons gamma, bêta et X.

13. Procédé selon la revendication 1, 3 ou 6, dans lequel la source de rayonnement est un rayonnement gamma, utilisé à une dose supérieure à environ 1 Mrad.

14. Procédé selon la revendication 1, 3 ou 6, dans lequel le matériau anti-microbien en cours d'irradiation est orienté sensiblement perpendiculairement au rayonnement incident.

15. Procédé selon l'une des revendications 1, 3 ou 6, dans lequel le matériau est placé adjacent à un matériau diélectrique au cours de l'irradiation.

16. Procédé selon la revendication 1, 3 ou 6, dans lequel le matériau est pris en sandwich entre des surfaces d'oxyde de silicium au cours de l'irradiation.

17. Matériau anti-microbien à grains fins, comprenant :

un ou plusieurs métaux anti-microbiens, ou alliages ou composés de tels métaux, sous la forme d'une poudre cristalline à grains fins, ayant une granulométrie inférieure à 200 nm, caractérisé par un désordre atomique suffisant pour que le matériau, en contact avec un alcool ou un électrolyte aqueux fournisse une libération prolongée d'atomes, d'ions, de molécules ou d'amas contenant au moins un métal à une concentration suffisante pour fournir un effet anti-microbien localisé, matériau dans lequel le métal anti-microbien est formé dans une matrice avec des atomes ou des molécules d'un matériau différent, le matériau différent étant choisi parmi les métaux biocompatibles inertes, l'oxygène, l'azote, l'hydrogène, le bore, le soufre, les halogènes, et les oxydes, nitrures, carbure, borures, sulfures et halogénures soit d'un métal anti-microbien, soit d'un métal inerte biocompatible, soit des deux.

18. Matériau anti-microbien selon la revendication 17, dans lequel ledit métal anti-microbien est choisi dans le groupe consistant en Ag, Au, Pt, Pd, Ir, Sn, Cu, Sb, Bi et Zn ou un alliage ou un composé de ceux-ci, et dans lequel le métal inerte biocompatible est choisi dans le groupe consistant en Ta, Ti, Nb, B, Hf, Zn, Mo, Si et Al.

19. Matériau anti-microbien selon la revendication 18, dans lequel le métal anti-microbien est choisi parmi Ag, Au ou Pd et dans lequel le métal inerte biocompatible est choisi parmi Ta, Ti ou Nb.

20. Matériau anti-microbien selon la revendication 17, comprenant de l'argent métal sensiblement pur, de l'oxyde d'argent et des atomes d'oxygène piégés ou absorbés.

21. Matériau anti-microbien selon les revendications 17, 18, 19 ou 20, sous la forme d'une poudre nanocristalline ayant une granulométrie inférieure à environ 20 nm, ou sous la forme d'une poudre à grains fins ayant une granulométrie inférieure à environ 140 nm.

22. Matériau anti-microbien selon la revendication 17, dans lequel le métal anti-microbien est l'argent, ou un alliage ou un composé d'argent, sous une forme cristalline, et dans lequel le matériau est caractérisé comme ayant un potentiel de repos positif lorsqu'il est mesuré par rapport à une électrode de référence au calomel saturée, dans de l'hydroxyde de potassium 1M et ayant un rapport entre sa température de recristallisation et sa température de fusion, exprimées en degrés K, $(T_{rec}/T_m)$, inférieur à 0,33 et qui, en contact avec un alcool ou un électrolyte aqueux, libère des atomes, des ions, des molécules ou des amas contenant de l'argent sur une base prolongée à une concentration suffisante pour fournir un effet anti-microbien localisé.

23. Matériau selon la revendication 22, dans lequel le matériau est en outre caractérisé en ce que le rapport entre sa température de recristallisation et sa température de fusion, exprimées en degrés K, ($T_{rec}/T_m$), est inférieur à environ 0,3.

**24.** Matériau selon la revendication 22, dans lequel le matériau est en outre caractérisé en ce qu'il a une température de recristallisation inférieure à environ 140°C.

**25.** Matériau selon la revendication 24, dans lequel le matériau est en outre caractérisé en ce qu'il a une granulométrie inférieure à environ 200 nm, ou dans lequel le matériau est en outre caractérisé en ce qu'il a une granulométrie inférieure à environ 140 nm, par exemple inférieure à 90 nm.

**26.** Matériau selon la revendication 25, sous la forme d'une poudre nanocristalline.

**27.** Matériau selon la revendication 26, sous la forme (a) d'argent métal sensiblement pur, ou (b) d'un mélange d'argent métal sensiblement pur et d'oxyde d'argent, ou (c) d'argent métal sensiblement pur et d'atomes ou de molécules d'oxygène absorbés, piégés, ou ayant réagis, et facultativement comprenant également de l'oxyde d'argent.

**28.** Procédé de production d'un matériau anti-microbien à grains fins, caractérisé par :

le dépôt d'un ou plusieurs métaux anti-microbiens dans une matrice avec des atomes ou des molécules d'un matériau différent, sous la forme d'une poudre, par dépôt en phase vapeur sur un substrat refroidi, pour fournir un matériau cristallin ayant un désordre atomique, tel que la poudre, en contact avec un alcool ou un électrolyte aqueux, fournit une libération prolongée d'ions, d'atomes, de molécules ou d'amas de l'un au moins des métaux anti-microbiens dans l'alcool ou l'électrolyte aqueux à une concentration suffisante pour fournir un effet anti-microbien localisé, procédé dans lequel le matériau différent est choisi dans le groupe consistant en les métaux biocompatibles inertes, l'oxygène, l'azote, l'hydrogène, le bore, le soufre, les halogènes, et les oxydes, nitrures, carbures, borures, sulfures et halogénures, soit d'un métal anti-microbien, soit d'un métal inerte biocompatible, soit des deux.

**29.** Procédé selon la revendication 28, dans lequel le métal anti-microbien est choisi dans le groupe consistant en Ag, Au, Pt, Pd, Ir, Sn, Cu, Sb, Bi et Zn ou les alliages ou composés de l'un ou plusieurs de ces métaux, et dans lequel le métal inerte biocompatible est choisi dans le groupe consistant en Ta, Ti, Nb, B, Hf, Zn, Mo, Si et Al ou les alliages ou composés de l'un ou plusieurs de ces métaux.

**30.** Procédé selon la revendication 29, dans lequel le métal anti-microbien est choisi parmi Ag, Au et Pd et dans lequel le métal inerte biocompatible est choisi parmi Ta, Ti et Nb.

**31.** Procédé selon la revendication 30, dans lequel de l'oxygène est inclus dans l'atmosphère de gaz de travail au cours du dépôt en phase vapeur de telle sorte que des atomes ou molécules d'oxygène sont piégés ou absorbés dans la matrice.

**32.** Procédé selon la revendication 31, dans lequel le métal anti-microbien qui est déposé est de l'argent métal sensiblement pur ou de l'oxyde d'argent et dans lequel de l'oxygène peut être inclus dans l'atmosphère du gaz de travail de telle sorte que le matériau déposé inclut de l'argent métal sensiblement pur et soit de l'oxyde d'argent, soit des atomes ou molécules d'oxygène piégés ou absorbés, soit les deux.

**33.** Procédé selon la revendication 30, 31 ou 32, dans lequel le matériau est déposé sous la forme d'une poudre à grains fins ayant une granulométrie inférieure à environ 200 nm, de préférence inférieure à 140 nm, par exemple une poudre nanocristalline ayant une granulométrie inférieure à environ 20 nm.

Fig. 2.

Fig. 1.